# EUROPEAN PATENT SPECIFICATION

(11) **EP 1 539 133 B1**
(45) Date of publication and mention of the grant of the patent: **23.08.2006**
(21) Application number: 03784064.2
(22) Date of filing: 23.07.2003
(51) Int. Cl.: A61K 31/33, C07D 519/00, A61P 31/04, C07D 513/00, C07D 471/00, C07D 498/00

(54) **AMINOCYCLOHEXENE QUINOLINES AND THEIR AZAISOSTERIC ANALOGUES WITH ANTIBACTERIAL ACTIVITY**
AMINOCYCLOHEXENCHINOLINE UND IHRE AZAISOSTERISCHEN ANALOGA MIT ANTIBAKTERIELLER WIRKUNG
MEDICAMENTS

(30) Priority: 25.07.2002 GB 0217294
(43) Date of publication of application: 15.06.2005
(73) Proprietor: GLAXO GROUP LIMITED, Greenford, Middlesex UB6 0NN (GB)
(72) Inventor: DAVIES, David Thomas, GlaxoSmithKline, Harlow, Essex CM19 5AW (GB); ELDER, John Stephen, GlaxoSmithKline, Harlow, Essex CM19 5AW (GB); FORREST, Andrew Keith, GlaxoSmithKline, Harlow, Essex M19 5AW (GB); JARVEST, Richard , GlaxoSmithKline,Med. Res.Centre, Stevenage, Hertfordshire SG1 2NY (GB); PEARSON, Neil David, GlaxoSmithKline, Harlow, Essex CM19 5AW (GB); SHEPPARD, Robert John, GlaxoSmithKline, Harlow, Essex CM19 5AW (GB)
(74) Representative: Valentine, Jill Barbara
(86) International application number: PCT/EP2003/008153
(87) International publication number: WO 2004/014361

(56) References cited:
- WO-A-00/78748
- WO-A-01/07432
- WO-A-01/07433
- WO-A-02/08224
- WO-A-02/056882

## Description

This invention relates to novel compounds, compositions containing them and their use as antibacterials.

WO099/37635, WO00/21948, WO00/21952, WO00/43383, WO00/78748, WO01/07433, WO01/07432, WO02/08224, WO02/24684, WO02/50040, WO02/50061, WO01/25227 and W002/O40474 disclose quinoline and naphthyridine derivatives having antibacterial activity.

This invention provides a compound of formula (I) or a pharmaceutically acceptable derivative thereof: 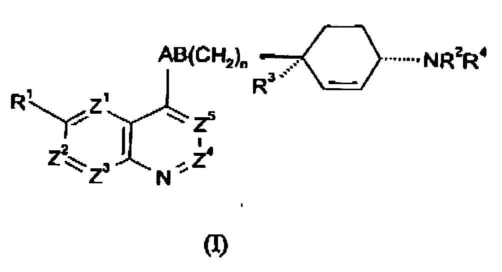 wherein:
one of Z¹, Z², Z³, Z⁴ and Z⁵ is N, one is CR^{1a} and the remainder are CH, or one of Z¹, Z², Z³, Z⁴ and Z⁵ is CR^{1a} and the remainder are CH;
R¹ and R^{1a} are independently selected from hydrogen; hydroxy; (C₁₋₆) alkoxy optionally substituted by (C₁₋₆)alkoxy, amino, piperidyl, guanidino or amidino any of which is optionally N-substituted by one or two (C₁₋₆)alkyl, acyl or (C₁₋₆)alkylsulphonyl groups, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆)alkyl; halogen; (C₁₋₆)alkyl; (C₁₋₆)alkylthio; trifluromethyl; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups, or when Z¹ is CR^{1a}, R¹ and R^{1a} may together represent (C₁₋₂)alkylenedioxy, or when Z⁵ is CR^{1a}, R^{1a} may instead be, cyano, hydroxymethyl or carboxy,
   provided that when Z¹, Z², Z³, Z⁴ and Z⁵ are CR^{1a} or CH, then R¹ is not hydrogen;
R² is hydrogen, or (C₁₋₄)alkyl or (C₂₋₄)alkenyl optionally substituted with 1 to 3 groups selected from:
   amino optionally substituted by one or two (C₁₋₄)alkyl groups; carboxy; (C₁₋₄) alkoxycarbonyl; (C ₁₋₄)alkylcarbonyl; (C₂₋₄)alkenyloxycarbonyl; (C₂₋₄) alkenylcarbonyl; aminocarbonyl wherein the amino group is optionally substituted by hydroxy, (C₁₋₄)alkyl, hydroxy(C₁₋₄)alkyl, aminocarbonyl(C₁₋₄)alkyl, (C₂₋₄)alkenyl, (C₁₋₄)alkylsulphonyl, trifluoromethylsulphonyl, (C₂₋₄)alkenylsulphonyl, (C₁₋₄) alkoxycarbonyl, (C₁₋₄)alkylcarbonyl, (C₂₋₄)alkenyloxycarbonyl or (C₂₋₄) alkenylcarbonyl; cyano; tetrazolyl; 2-oxo-oxazolidinyl optionally substituted by R¹⁰; 3-hydroxy-3-cyclobatene-1,2-dione-4-yl; 2,4-thiazolidinedione-5-yl; tetrazol-5-ylaminocarbonyl; 1,2,4-triazol-5-yl optionally substituted by R¹⁰; 5-oxo-1,2,4-oxadiazol-3-yl; halogen; (C₁₋₄)alkylthio; trifluoromethyl; hydroxy optionally substituted by (C₁₋₄) alkyl, (C₂₋₄)alkenyl, (C₁₋₄)alkoxycarbonyl, (C₁₋₄)alkylcarbonyl, (C₂₋₄) alkenyloxycarbonyl, (C₂₋₄)alkenylcarbonyl; oxo; (C₁₋₄)alkylsulphonyl; (C₂₋₄) alkenylsulphonyl; or (C₁₋₄)aminosulphonyl wherein the amino group is optionally substituted by (C₁₋₄)alkyl or (C₂₋₄)alkenyl;
R³ is hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆) alkylcarbonyl or (C₂₋₆)alkenylcarbonyl;
R¹⁰ is selected from (C₁₋₄)alkyl and (C₂₋₄)alkenyl either of which may be optionally substituted by a group R¹² as defined above; carboxy; aminocarbonyl wherein the amino group is optionally substituted by hydroxy, (C₁₋₆)alkyl, (C₂₋₆)alkenyl, (C₁₋₆) alkylsulphonyl, trifluoromethylsulphonyl, (C₂₋₆)alkenylsulphonyl, (C₁₋₆) alkoxycarbonyl, (C₁₋₆)alkylcarbonyl, (C₂₋₆)alkenyloxycarbonyl or (C₂₋₆) alkenylcarbonyl and optionally further substituted by (C₁₋₆)alkyl or (C₂₋₆)alkenyl; (C₁₋₆)alkylsulphonyl; trifluoroxnethylsulphonyl; (C₂₋₆)alkenylsulphonyl; (C₁₋₆) alkoxycarbonyl; (C₁₋₆)alkylcarbonyl; (C₂₋₆)alkenyloxycorbonyl; and (C₂₋₆) alkenylcarbonyl;
R⁴ is a group -CH₂-R⁵₁ in which R⁵₁ is selected from:
   (C₄₋₈)alkyl; hydroxy(C₄₋₈)alkyl; (C₁₋₄)alkoxy(C₄₋₈)alkyl; (C₁₋₄) alkanoyloxy(C₄₋₈)alkyl; (C₃₋₈)cycloalkyl(C₄₋₈)alkyl; hydroxy-, (C₁₋₆)alkoxy- or (C₁₋₆) alkanoyloxy-(C₃₋₈)cycloalkyl(C₄₋₈)alkyl; cyano(C₄₋₈)alkyl; (C₄₋₈)alkenyl; (C₄₋₈) alkynyl; tetrahydrofuryl; mono- or di-(C₁₋₆)alkylamino(C₄₋₈)alkyl; acylamino(C₄₋₈) ₈)alkyl; (C₁₋₆)alkyl- or acyl-aminocarbonyl(C₄₋₈)alkyl; mono- or di- (C₁₋₆) alkylamino(hydroxy) (C₄₋₈)alkyl; or
R⁴ is a group -U-R⁵₂ where R⁵₂ is an optionally substituted bicyclic carbocyclic or heterocyclic ring system (A): 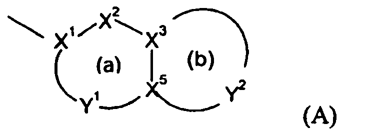
containing up to four heteroatoms in each ring in which
   at least one of rings (a) and (b) is aromatic;
   X¹ is C or N when part of an aromatic ring or CR¹⁴ when part of a non aromatic ring;
   X² is N, NR¹³, O, S(O)ₓ, CO or CR¹⁴ when part of an aromatic or non-aromatic ring or may in addition be CR¹⁴R¹⁵ when part of a non aromatic ring;
   X³ and X⁵ are independently N or C;
   Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non aromatic ring,
   Y² is a 2 to 6 atom linker group, each atom of Y² being independently selected from N, NR¹³, O, S(O)ₓ, CO and CR¹⁴ when part of an aromatic or non-aromatic ring or may additionally be CR¹⁴R¹⁵ when part of a non aromatic ring;
   each of R¹⁴ and R¹⁵ is independently selected from: H; (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄) alkoxycarbonyl; formyl; (C₁₋₄)alkylcarbonyl; (C₂₋₄)alkenyloxycarbonyl; (C₂₋₄) alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄) alkylsulphonyl; (C₂₋₄)alkenylsulphonyl; or aminosulphonyl wherein the amino group is optionally substituted by (C₁₋₄)alkyl or (C₂₋₄)alkenyl; aryl; aryl(C₁₋₄)alkyl; aryl(C₁₋₄) alkoxy;
   each R¹³ is independently H; trifluoromethyl; (C₁₋₄)alkyl optionally substituted by hydroxy, carboxy, (C₁₋₆)alkoxycarbonyl, (C₁₋₆)alkoxy, (C₁₋₆)alkylthio, halo or trifluoromethyl; (C₂₋₄)alkenyl; aryl; aryl (C ₁₋₄)alkyl; arylcarbonyl; heteroarylcarbonyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄) alkoxycarbonyl, (C₁₋₄)alkylcarbonyl, (C₂₋₄)alkenyloxycarbonyl, (C₂₋₄) ₄)alkenylcarbonyl, (C₁₋₄)alkyl or (C₂₋₄)alkenyl and optionally further substituted by (C₁₋₄) alkyl or (C₂₋₄)alkenyl;
   each x is independently 0, 1 or 2;
U is CO, SO₂ or CH₂; or
R⁴ is a group -X^{1a}-X^{2a}-X^{3a}-X^{4a} in which:
   X^{1a} is CH₂, CO or SO₂;
   X^{2a} is CR^{14a}R^{15a};
   X^{3a} is NR^{13a}, O, S, SO₂ or CR^{14a}R^{15a}; wherein:
   each of R^{14a} and R^{15a} is independently selected from the groups listed above for R¹⁴ and R¹⁵, provided that R^{14a} and R^{15a} on the same carbon atom are not both selected from optionally substituted hydroxy and optionally substituted amino; or
   R^{14a} and R^{15a} together represent oxo;
   R^{13a} is hydrogen; trifluoromethyl; (C₁₋₆)alkyl; (C₂₋₆)alkenyl; (C₁₋₆) alkoxycarbonyl; (C₁₋₆)alkylcarbonyl; or aminocarbonyl wherein the amino group is optionally substituted by (C₁₋₆)alkoxycarbonyl, (C₁₋₆)alkylcarbonyl, (C₂₋₆) alkenyloxycarbonyl, (C₂₋₆)alkenylcarbonyl, (C₁₋₆)alkyl or (C₂₋₆)alkenyl and optionally further substituted by (C ₁₋₆)alkyl or (C₂₋₆)alkenyl; or
   two R^{14a} groups or an R^{13a} and an R^{14a} group on adjacent atoms together represent a bond and the remaining R^{13a}, R^{14a} and R^{15a} groups are as above defined; or
   two R^{14a} groups and two R^{15a} groups on adjacent atoms together represent bonds such that X^{2a} and X^{3a} is triple bonded;
   X^{4a} is phenyl or C or N linked monocyclic aromatic 5- or 6-membered heterocycle containing up to four heteroatoms selected from O, S and N and: optionally C-substituted by up to three groups selected from (C₁₋₄)alkylthio; halo; carboxy(C₁₋₄) alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; aryl, aryl(C₁₋₄)alkyl or aryl(C₁₋₄)alkoxy; and
optionally N substituted by trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl(C₁₋₄)alkyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C ₁₋₄)alkoxycarbonyl, (C₁₋₄)alkylcarbonyl, (C₂₋₄)alkenyloxycarbonyl, (C_{2- 4})alkenylcarbonyl, (C₁₋₄)alkyl or (C₂₋₄)alkenyl and optionally further substituted by (C₁₋₄)alkyl or (C₂₋₄)alkenyl;
n is 0 or 1 and AB is NR¹¹CO, CONR¹¹, CO-CR⁸R⁹, CR⁶R⁷-CO, O-CR⁸R⁹, CR⁶R⁷⁻O , NHR¹¹-CR⁸R⁹, CR⁶R⁷- NHR¹¹, NR¹¹SO₂, CR⁶R⁷-SO₂ or CR⁶R⁷-CR⁸R⁹, provided that if n=0, B is not NR¹¹, O or SO₂,
and provided that R⁶ and R⁷, and R⁸ and R⁹ are not both optionally substituted hydroxy or amino;
and wherein:
each of R⁶, R⁷, R⁸ and R⁹ is independently selected from: H; (C₁₋₆)alkoxy; (C₁₋₆)alkylthio; halo; trifluoromethyl; azido; (C₁₋₆)alkyl; (C₂₋₆)alkenyl; (C₁₋₆)alkoxycarbonyl; (C₁₋₆)alkylcarbonyl; (C₂₋₆)alkenyloxycarbonyl; (C₂₋₆)alkenylearbonyl; hydroxy, amino or aminocarbonyl optionally substituted as for corresponding substituents in R³; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl;
or R⁶ and R⁸ together represent a bond and R⁷ and R⁹ are as above defined;
in optionally substituted amino the amino group is optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C ₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl;
in optionally substituted aminocarbonyl the amino group is optionally substituted by (C₁₋₆)alkyl, hydroxy(C₁₋₆)alkyl, aminocarbonyl(C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl;
and each R¹¹ is independently H; trifluoromethyl; (C₁₋₆)alkyl; (C₂₋₆)alkenyl; (C₁₋₆)alkoxycarbonyl; (C₁₋₆)alkylcarbonyl; or aminocarbonyl wherein the amino group is optionally substituted by (C₁₋₆)alkoxycarbonyl, (C ₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl;
or where one of R⁶, R⁷, R⁸ or R⁹ contains a carboxy group they may together with R³ form a cyclic ester linkage.

The invention also provides the use of a compound of formula (I) or a pharmaceutically acceptable derivative thereof in the manufacture of a medicament for use in the treatment of bacterial infections in mammals.

The invention also provides a pharmaceutical composition, in particular for use in the treatment of bacterial infections in mammals, comprising a compound of formula (I), or a pharmaceutically acceptable derivative thereof, and a pharmaceutically acceptable carrier.

The invention further provides a method of treatment of bacterial infections in mammals, particularly in man, which method comprises the administration to a mammal in need of such treatment of an effective amount of a a compound of formula (I), or a pharmaceutically acceptable derivative thereof.

Preferably Z⁵ is CH, Z³ is CH or CF, Z¹ is CH or C-OCH₃ and Z² and Z⁴ are each CH, or Z¹ is N, Z³ is CH or CF and Z², Z⁴ and Z⁵ are each CH

Compounds of formula (I) in which the enantiomeric configuration of the substituted cyclohexene group corresponds to that of enantiomer E2 described in the Examples are preferred.

When R¹ or R^{1a} is substituted alkoxy it is preferably (C₂₋₆)alkoxy substitituted by optionally N-substituted amino, guanidino or amidino, or (C ₁₋₆)alkoxy substituted by piperidyl. Suitable examples of R¹ alkoxy include methoxy, trifluoromethoxy, n-propyloxy, i-butyloxy, aminoethyloxy, aminopropyloxy, aminobutyloxy, aminopentyloxy, guanidinopropyloxy, piperidin-4-ylmethyloxy or 2-aminocarbonylprop-2-oxy. Preferably R¹ is methoxy, amino(C₃₋₅)alkyloxy, guanidino(C₃₋₅)alkyloxy, piperidyl(C₃₋₅)alkyloxy, nitro or fluoro.

Preferably R¹ and R^{1a} are independently methoxy, amino(C₃₋₅)alkyloxy, guanidino(C₃₋₅)alkyloxy, piperidyl(C₃₋₅)alkyloxy, nitro or fluoro; more preferably methoxy, fluoro, amino(C₃₋₅)alkyloxy or guanidino(C₃₋₅)alkyloxy. Preferably R^{1a} is H, methoxy or F. Most preferably R¹ is methoxy or fluoro and R^{1a} is H or when Z³ is CR^{1a} it may be C-F.

When Z⁵ is CR^{1a}, R^{1a} is preferably hydrogen, cyano, hydroxymethyl or carboxy, most preferably hydrogen.

R² is preferably hydrogen; (C₁₋₄)alkyl substituted with carboxy, optionally substituted hydroxy, optionally substituted aminocarbonyl, optionally substituted amino or (C₁₋₄)alkoxycarbonyl; or (C₂₋₄)alkenyl substituted with (C₁₋₄)alkoxycarbonyl or carboxy. More preferred groups for R² are hydrogen, carboxymethyl, hydroxyethyl, aminocarbonylmethyl, ethoxycarbonylmethyl, ethoxycarbonylallyl and carboxyallyl, most preferably hydrogen.

R³ is preferably hydroxy or (C₁₋₆) alkoxy, most preferably hydroxy.

When R³ and R⁶, R⁷, R⁸ or R⁹ together form a cyclic ester linkage, it is preferred that the resulting ring is 5-7 membered. It is further preferred that the group A or B which does not form the ester or amide linkage is CH₂.

When A is CH(OH) the R-stereochemistry is preferred.

Preferably A is NH, NCH₃, CH₂, CHOH, CH(NH₂), C(Me)(OH) or CH(Me).

Preferably B is CH₂ or CO.

Preferably n=0.

Most preferably:
n is 0 and either A is CHOH, CH₂ and B is CH₂ or A is NH and B is CO.

Preferably R¹¹ is hydrogen or (C₁₋₄)alkyl e.g. methyl, more preferably hydrogen.

When R⁴ is CH₂R⁵₁, preferably R⁵₁ is (C₆₋₈)alkyl.

When R⁴ is a group -X^{1a}-X^{2a}-X^{3a}-X^{4a:}

X^{1a} is preferably CH₂.

X^{2a} is preferably CH₂ or together with X^{3a} forms a CH=CH or C=C group.

X^{3a} is preferably CH₂, O, S or NH, or together with X^{2a} forms a CH=CH or C≡C group.

Preferred linker groups -X^{1a}-X^{2a}-X^{3a}- include -(CH₂)₂-O-, -CH₂-CH=CH-, - (CH₂)₃-, -(CH₂)₂-NH- or -CH₂CONH-.

Monocyclic aromatic heterocyclic groups for X^{4a} include pyridyl, pyrazinyl, pyrimidinyl, triazolyl, tetrazolyl, thienyl, isoimidazolyl, thiazolyl, furanyl and imidazolyl, 2H-pyridazone, 1H-pyrid-2-one. Preferred aromatic heterocyclic groups include pyrid-2-yl, pyrid-3-yl, thiazole-2-yl, pyrimidin-2-yl, pyrimidin-5-yl and fur-2-yl.

Preferred substituents on heterocyclic X^{4a} include halo especially fluoro, trifluoromethyl and nitro.

Preferred substituents on phenyl X^{4a} include halo, especially fluoro, nitro, cyano, trifluoromethyl, methyl, methoxycarbonyl and methylcarbonylamino.

Preferably X^{4a} is 2-pyridyl, 3-fluorophenyl, 3,5-difluorophenyl or thiazol-2-yl.

Preferably R⁴ is -U-R⁵₂.

The group -U- is preferably -CH₂-.

Preferably R⁵₂ is an aromatic heterocyclic ring (A) having 8-11 ring atoms including 2-4 heteroatoms of which at least one is N or NR¹³.

Alternatively and preferably the heterocyclic ring (A) has ring (a) aromatic selected from optionally substituted benzo and pyrido and ring (b) non-aromatic and Y² has 3-5 atoms including NR¹³, O or S bonded to X⁵ and NHCO bonded via N to X³, or O bonded to X³. Examples of rings (A) include optionally substituted:

### (a) and (b) aromatic

1H-pyrrolo[2,3-b]-pyridin-2-yl, 1H-pyrrolo[3,2-b]-pyridin-2-yl, 3H-imidazo[4,5-b]-pyrid-2-yl, 3H-quinazolin-4-one-2-yl, benzimidazol-2-yl, benzo[1,2,3]-thiadiazol-5-yl, benzo[1,2,5]-oxadiazol-5-yl, benzofur-2-yl, benzothiazol-2-yl, benzo[b]thiophen-2-yl, benzoxazol-2-yl, chromen-4-one-3-yl, imidazo[1,2-a]pyridin-2-yl, imidazo-[1,2-a]-pyrimidin-2-yl, indol-2-yl, indol-6-yl, isoquinolin-3-yl, [1,8]-naphthyridine-3-yl, oxazolo[4,5-b]-pyridin-2-yl, quinolin-2-yl, quinolin-3-yl, quinoxalin-2-yl, indan-2-yl, naphthalen-2-yl, 1,3-dioxo-isoindol-2yl, benzimidazol-2-yl, benzothiophen-2-yl, 1H-benzotriazol-5-yl, 1H-indol-5-yl, 3H-benzooxazol-2-one-6-yl, 3H-benzooxazol-2-thione-6-yl, 3H-benzothiazol-2-one-5-yl, 3H-quinazolin-4-one-2-yl, 3H-quinazolin-4-one-6-yl, 4-oxo-4H-pyrido[1,2-a]pyrimidin-3 -yl, benzo[ 1,2,3]thiadiazol-6-yl, benzo[1,2,5]thiadiazol-5-yl, benzo[1,4]oxazin-2-one-3-yl, benzothiazol-5-yl, benzothiazol-6-yl, cinnolin-3-yl, imidazo[1,2-a]pyridazin-2-yl, imidazo[1,2-b]pyridazin-2-yl, pyrazolo[1,5-a]pyrazin-2-yl, pyrazolo[1,5-a]pyridin-2-yl, pyrazolo[1,5-a]pyrimidin-6-yl, pyrazolo[5,1-c][1,2,4]triazin-3-yl, pyrido[1,2-a]pyrimdin-4-one-2-yl, pyrido[1,2-a]pyrimidin-4-one-3-yl, quinazolin-2-yl, quinoxalin-6-yl, thiazolo[3,2-a]pyrimidin-5-one-7-yl, thiazolo[5,4-b]pyridin-2-yl, thieno[3,2-b]pyridin-6-yl, thiazolo[5,4-b]pyridin-6-yl, 4-oxo-4H-pyrido[1,2-a]pyrimidin-2-yl, 1-oxo-1,2-dihydro-isoquinolin-3-yl, thiazolo[4,5-b]pyridin-5-yl, [1,2,3]thiadiazolo[5,4-b]pyridin-6-yl, 2H-isoquinolin-1-one-3-yl

### (a) is non aromatic

(2S)-2,3-dihydro-1H-indol-2-yl, (2S)-2,3-dihydro-benzo[1,4]dioxine-2-yl, 3-(R,S)-3,4-dihydro-2H-benzo[1,4]thiazin-3-yl, 3-(R)-2,3-dihydro-[1,4]dioxino[2,3-b]pyridin-3-yl, 3-(S)-2,3-dihydro-[I,4]dioxino[2,3-b]pyridin-3-yl, 2,3-dihydro-benzo[1,4]dioxan-2-yl, 3-substituted-3H-quinazolin-4-one-2-yl,

### (b) is non aromatic

1,1,3-trioxo-1,2,3,4-tetrahydro-1 *l*⁶-benzo[1,4] thiazin-6-yl, benzo[1,3]dioxol-5-yl, 4H-benzo[1 ,4]oxazin-3-one-6-yl, 2,3-dihydro-benzo[1,4]dioxin-6-yl, 2-oxo-2,3-dihydro-benzooxazol-6-yl, 4H-benzo[1,4]oxazin-3-one-6-yl (3-oxo-3,4-dihydro-2H-benzo[1,4]oxazin-6-yl), 4H-benzo[1,4]thiazin-3-one-6-yl (3-oxo-3,4-dihydro-2H-benzo[1,4]thiazin-6-yl), 4H-benzo[1,4]oxazin-3-one-7-yl, 4-oxo-2,3,4,5-tetrahydro-benzo[b][1,4]thiazepine-7-yl, 5-oxo-2,3-dihydro-5H-thiazolo[3,2-a]pyrimidin-6-yl, benzo[1,3]dioxol-5-yl, 2-oxo-2,3-dihydro-1H-pyrido[2,3-b][1,4]thiazin-7-yl, 2-oxo-2,3-dihydro-1H-pyrido[3,4-b][1,4]thiazin-7-yl, 3-oxo-3,4-dihydro-2H-pyrido[3,2-b][1,4]thiazin-6-yl, 2,3-dihydro-[1,4]dioxino[2,3-b]pyridin-6-yl, 2,3-dihydro-[1,4]dioxino[2,3-c]pyridin-7-yl, 2,3-dihydro-[1,4]dioxino[2,3-b]pyridin-7-yl, 6,7-dihydro-[1,4]dioxino[2,3-d]pyrimidin-2-yl, 3-oxo-3,4-dihydro-2H-pyrido[3,2-b][1,4]oxazin-6-yl, 2-oxo-2,3-dihydro-1H-pyrido[3,4-b][1,4]oxazin-7-yl, 2-oxo-2,3-dihydro-1H-pyrido[2,3-b][1,4]oxazin-7-yl, 6-oxo-6,7-dihydro-5H-8-thia-1,2,5-triaza-naphthalen-3-yl, 3,4-dihydro-2H-benzo[1,4]oxazin-6-yl, 3,4-dihydro-2H-benzo[1,4]oxazin-6-yl, 3-substituted-3H-benzooxazol-2-one-6-yl, 3-substituted-3H-benzooxazole-2-thione-6-yl, 3-substituted-3H-benzothiazol-2-one-6-yl, 2,3-dihydro-1H-pyrido[2,3-b][1,4]thfazin-7-yl, 3,4-dihydro-2H-benzo[1,4]thiazin-6-yl, 3,4-dihydro-1H-quinolin-2-one-7-yl, 3,4-dihydro-1H-quinoxalin-2-one-7-yl, 6,7-dihydro-4H-pyrazolo[1,5-a]pyrimidin-5-one-2-yl, 5,6,7,8-tetrahydro-[1,8]naphthyridin-2-yl, 2-oxo-3,4-dihydro-1*H*-[1,8]naphthyridin-6-yl.

R¹³ is preferably H if in ring (a) or in addition (C₁₋₄)alkyl such as methyl or isopropyl when in ring (b). More preferably, in ring (b) R¹³ is H when NR¹³ is bonded to X³ and (C₁₋₄)alkyl when NR¹³ is bonded to X⁵.

R¹⁴ and R¹⁵ are preferably independently selected from hydrogen, halo, hydroxy, (C₁₋₄) alkyl, (C₁₋₄)alkoxy, trifluoromethoxy, nitro, cyano, aryl(C₁₋₄)alkoxy and (C₁₋₄)alkylsulphonyl.

More preferably R¹⁵ is hydrogen.

More preferably each R¹⁴ is selected from hydrogen, chloro, fluoro, hydroxy, methyl, methoxy, trifluoromethoxy, benzyloxy, nitro, cyano and methylsulphonyl. Most preferably R¹⁴ is selected from hydrogen, hydroxy, fluorine or nitro. Preferably 0-3 groups R¹⁴ are substituents other than hydrogen.

Most preferred groups R⁵₂ include:
[1,2,3]thiadiazolo[5,4-b]pyridin-6-yl
1H-pyrrolo[2,3-b]pyridin-2-yl
2,3-dihydro-[1,4]dioxino[2,3-b]pyridin-6-yl
2,3-dihydro-[1,4]dioxino[2,3-b]pyridin-7-yl
2,3-dihydro-[1,4]dioxino[2,3-c]pyridin-7-yl
2,3-dihydro-benzo[1,4]dioxin-6-yl
2-oxo-2,3-dihydro-1H-pyrido[2,3-b][1,4]oxazin-7-yl
2-oxo-2,3-dihydro-1H-pyrido[2,3-b][1,4]thiazin-7-yl
3,4-dihydro-2H-benzo[1,4]oxazin-6-yl
3-methyl-2-oxo-2,3-dihydro-benzooxazol-6-yl
3-oxo-3,4-dihydro-2H-benzo[1,4]oxazin-6-yl
3-oxo-3,4-dihydro-2H-pyrido[3,2-b][1,4]oxazin-6-yl
4H-benzo[1,4]thiazin-3-one-6-yl
4-oxo-4H-pyrido[1,2-a]pyrimidin-2-yl
6-nitro-benzo[1,3]dioxol-5-yl
7-fluoro-3-oxo-3,4-dihydro-2H-benzo[1,4]oxazin-6-yl
8-hydroxy-1-oxo-1,2-dihydro-isoquinolin-3-yl
8-hydroxyquinolin-2-yl
benzo[ 1,2,3]thiadiazol-5-yl
benzo[ 1,2,5]thiadiazol-5-yl
benzothiazol-5-yl
thiazolo-[5,4-b]pyridin-6-yl
3-oxo-3,4-dihydro-2*H*-pyrido[3,2-*b*][1,4]thiazin-6-yl
7-chloro-3-oxo-3,4-dihydro-2*H*-pyrido[3,2-*b*][1,4]thiazin-6-yl
7-fluoro-3-oxo-3,4-dihydro-2*H*-pyrido[3,2-*b*][1,4]thiazin-6-yl
2-oxo-2,3-dihydro-1*H*-pyrido[3,4-*b*][1,4]thiazin-7-yl
especially
benzo[1,2,5]thiadiazol-5-yl
4H-benzo[1,4]thiazin-3-one-6-yl
2,3-dihydro-benzo[1,4]dioxin-6-yl
benzo[ 1,2,3]thiadiazol-5-yl
3-oxo-3,4-dihydro-2H-benzo[1,4]oxazin-6-yl
7-fluoro-3-oxo-3,4-dihydro-2H-benzo[1,4]oxazin-6-yl
2-oxo-2,3-dihydro-1H-pyrido[2,3-b][1,4]thiazin-7-yl
2,3-dihydro-[1,4]dioxino[2,3-c]pyridin-7-yl
3-oxo-3,4-dihydro-2H-pyrido[3,2-b][1,4]oxazin-6-yl
[ 1,2,3]thiadiazolo[5,4-b]pyridin-6-yl
3-oxo-3,4-dihydro-2*H*-pyrido[3,2-*b*][1,4]thiazin-6-yl
7-chloro-3-oxo-3,4-dihydro-2*H*-pyrido[3,2-*b*][1,4]thiazin-6-yl
7-fluoro-3-oxo-3,4-dihydro-2*H*-pyrido[3,2-*b*][1,4]thiazin-6-yl
2-oxo-2,3-dihydro-1*H*-pyrido[3,4-*b*][1,4]thiazin-7-yl
most especially
3-oxo-3,4-dihydro-2*H*-pyrido[3,2-*b*][1,4]thiazin-6-yl
3-oxo-3,4-dihydro-2*H*-pyrido[3,2-*b*][1,4]oxazin-6-yl .
2,3-dihydro-[1,4]dioxino[2,3-c]pyridin-7-yl.

When used herein, the term "alkyl" includes groups having straight and branched chains, for instance, methyl, ethyl, n-propyl, iso-propyl, n-butyl, iso-butyl, sec-butyl, t-butyl, pentyl and hexyl. The term 'alkenyl' should be interpreted accordingly.

Halo or halogen includes fluoro, chloro, bromo and iodo.

Haloalkyl moieties include 1-3 halogen atoms.

Unless otherwise defined, the term 'heterocyclic' as used herein includes aromatic and non-aromatic, single and fused, rings suitably containing up to four hetero-atoms in each ring selected from oxygen, nitrogen and sulphur, which rings may be unsubstituted or C-substituted by, for example, up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C ₁₋₄)alkylcarbonyloxy; (C ₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; optionally substituted aryl, aryl(C₁₋₄)alkyl or aryl(C₁₋₄)alkoxy and oxo groups. Each heterocyclic ring suitably has from 4 to 7, preferably 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

Where an amino group forms part of a single or fused non-aromatic heterocyclic ring as defined above suitable optional substituents in such substituted amino groups include H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl (C ₁₋₄)alkyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl..

When used herein the term 'aryl', includes phenyl and naphthyl, each optionally substituted with up to five, preferably up to three, groups selected from(C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C ₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano, carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; phenyl, phenyl(C₁₋₄)alkyl or phenyl(C₁₋₄)alkoxy.

The term 'acyl' includes (C₁₋₆)alkoxycarbonyl, formyl or (C₁₋₆) alkylcarbonyl groups.

Some of the compounds of this invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

Since the compounds of formula (I) are intended for use in pharmaceutical compositions it will readily be understood that they are each provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 59% of a compound of the formula (I) or pharmaceutically acceptable derivative thereof.

Pharmaceutically acceptable derivatives of the above-mentioned compounds of formula (I) include the free base form or their acid addition or quaternary ammonium salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids. Compounds of formula (I) may also be prepared as the N-oxide. Compounds of formula (I) having a free carboxy group may also be prepared as an *in vivo* hydrolysable ester. The invention extends to all such derivatives.

Examples of suitable pharmaceutically acceptable *in vivo* hydrolysable ester-forming groups include those forming esters which break down readily in the human body to leave the parent acid or its salt. Suitable groups of this type include those of part formulae (i), (ii), (iii), (iv) and (v):

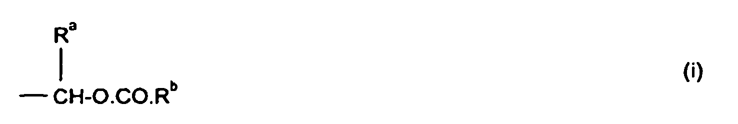

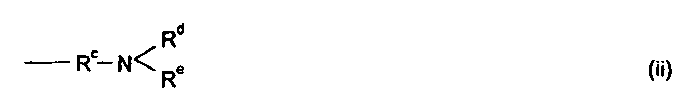

―CH₂―OR^{f} (iii)

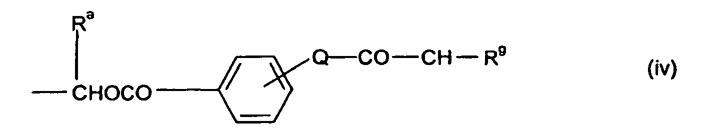

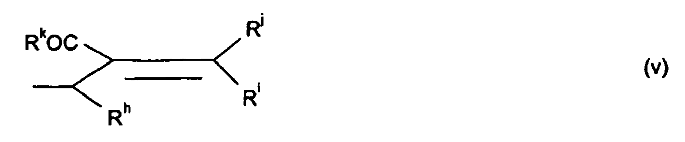

wherein R^{a} is hydrogen, (C₁₋₆) alkyl, (C₃₋₇) cycloalkyl, methyl, or phenyl, R^{b} is (C₁₋₆) alkyl, (C₁₋₆) alkoxy, phenyl, benzyl, (C₃₋₇) cycloalkyl, (C₃₋₇) cycloalkyloxy, (C₁₋₆) alkyl (C₃₋₇) cycloalkyl, 1-amino (C₁₋₆) alkyl, or 1-(C₁₋₆ alkyl)amino (C₁₋₆) alkyl; or R^{a} and R^{b} together form a 1,2-phenylene group optionally substituted by one or two methoxy groups; R^{c} represents (C₁₋₆) alkylene optionally substituted with a methyl or ethyl group and R^{d} and R^{e} independently represent (C₁₋₆) alkyl; R^{f} represents (C₁₋₆) alkyl; R^{g} represents hydrogen or phenyl optionally substituted by up to three groups selected from halogen, (C₁₋₆) alkyl, or (C₁₋₆) alkoxy; Q is oxygen or NH; R^{h} is hydrogen or (C₁₋₆) alkyl; Rⁱ is hydrogen, (C₁₋₆) alkyl optionally substituted by halogen, (C₂₋₆) alkenyl, (C₁₋₆) alkoxycarbonyl, aryl or heteroaryl; or R^{h} and Rⁱ together form (C₁₋₆) alkylene; R^{j} represents hydrogen, (C₁₋₆) alkyl or (C₁₋₆) alkoxycarbonyl; and R^{k} represents (C₁₋₈) alkyl, (C₁₋₈) alkoxy, (C₁₋₆) alkoxy(C₁₋₆)alkoxy or aryl.

Examples of suitable *in vivo* hydrolysable ester groups include, for example, acyloxy(C₁₋₆)alkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)carbonyloxymethyl; (C₁₋₆)alkoxycarbonyloxy(C₁₋₆)alkyl groups, such as ethoxycarbonyloxymethyl, α-ethoxycarbonyloxyethyl and propoxycarbonyloxyethyl; di(C₁₋₆)alkylamino(C₁₋₆)alkyl especially di(C₁₋₄)alkylamino(C₁₋₄)alkyl groups such as dimethylaminomethyl, dimethylaminoethyl, diethylaminomethyl or diethylaminoethyl; 2-((C₁₋₆)alkoxycarbonyl)-2-(C₂₋₆)alkenyl groups such as 2-(isobutoxycarbonyl)pent-2-enyl and 2-(ethoxycarbonyl)but-2-enyl; lactone groups such as phthalidyl and dimethoxyphthalidyl.

A further suitable pharmaceutically acceptable *in vivo* hydrolysable ester-forming group is that of the formula: 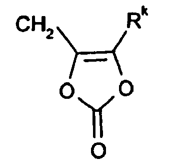
wherein R^{k} is hydrogen, C₁₋₆ alkyl or phenyl.
R is preferably hydrogen.

Certain of the above-mentioned compounds of formula (I) may exist in the form of optical isomers, e.g. diastereoisomers and mixtures of isomers in all ratios, e.g. racemic mixtures. The invention includes all such forms, in particular the pure isomeric forms. For examples the invention includes compound in which an A-B group CH(OH)-CH₂ is in either isomeric configuration the R-isomer is preferred. The different isomeric forms may be separated or resolved one from the other by conventional methods, or any given isomer may be obtained by conventional synthetic methods or by stereospecific or asymmetric syntheses..

In a further aspect of the invention there is provided a process for preparing compounds of formula (I), or a pharmaceutically acceptable derivative thereof, which process comprises reacting a compound of formula (IV) with a compound of formula (V): 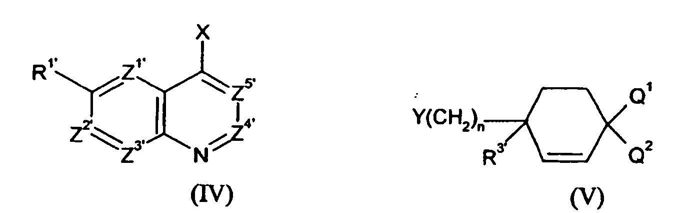 wherein n is as defined in formula (I); Z^{1'}, Z^{2'}, Z^{3'}, Z^{4'}, Z^{5'}, R^{1'} and R^{3'} are Z¹, Z², Z³, Z⁴, Z⁵, R¹ and R³ as defined in formula (I) or groups convertible thereto; Q¹ is NR^{2'}R^{4'} or a group convertible thereto wherein R^{2'} and R^{4'} are R² and R⁴ as defined in formula (I) or groups convertible thereto and Q² is H or R^{3'} or Q¹ and Q² together form an optionally protected oxo group;
and X and Y may be the following combinations:
(i) one of X and Y is CO₂R^{y} and the other is CH₂CO₂R^{x};
(ii) X is CHR⁶R⁷ and Y is C(=O)R⁹;
(iii) X is CR⁷=PR^{z}₃ and Y is C(=O)R⁹;
(iv) X is C(=O)R⁷ and Y is CR⁹=PR^{z}₃;
(v) one of Y and X is COW and the other is NHR^{11'};
(vi) X is NHR^{11'} and Y is C(=O)R⁸ or X is C(=O)R⁶ and Y is NHR^{11'};
(vii) X is NHR^{11'} and Y is CR⁸R⁹W;
(viii) X is W or OH and Y is CH₂OH;
(ix) X is NHR^{11'} and Y is SO₂W;
(x) one of X and Y is (CH₂)ₚ-W and the other is (CH₂)_{q}NHR¹¹', (CH₂)_{q}OH, (CH₂)_{q}SH or (CH₂)_{q}SCOR^{x} where p+q=1;
(xi) one of X and Y is OH and the other is -CH=N₂;
(xii) X is W and Y is CONHR¹¹;
(xiii) X is W and Y is -C≡CH followed by selective reduction of the intermediate -C≡C- group;
in which W is a leaving group, e.g. halo or imidazolyl; R^{x} and R^{y} are (C₁₋₆)alkyl; R^{z} is aryl or (C₁₋₆)alkyl; A' and NR^{11'} are A and NR¹¹ as defined in formula (I), or groups convertible thereto; and oxirane is: 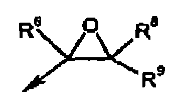 wherein R⁶, R⁸ and R⁹ are as defined in formula (I);
and thereafter optionally or as necessary converting Q ¹ and Q² to NR^{2'}R^{4'}; converting A', Z^{1'}, Z^{2'}, Z^{3'}, Z^{4'}, Z^{5'}, R^{1'}, R^{2'}, R^{3'}, R^{4'} and NR^{11'} to A, Z¹, Z², Z³, Z⁴, Z⁵, R¹, R², R³, R⁴ and NR¹¹; converting A-B to other A-B, interconverting R^{v}, R^{w}, R¹, R², R³ and/or R⁴, and/or forming a pharmaceutically acceptable derivative thereof.

Process variant (i) initially produces compounds of formula (I) wherein A-B is CO-CH₂ or CH₂-CO.

Process variant (ii) initially produces compounds of formula (I) wherein A-B is CR⁶R⁷-CR⁹OH.

Process variant (iii) and (iv) initially produce compounds of formula (I) wherein A-B is CR⁷=CR⁹.

Process variant (v) initially produces compounds of formula (I) where A-B is CO-NR¹¹ or NR¹¹-CO.

Process variant (vi) initially produces compounds of formula (I) wherein A-B is NR¹¹-CHR⁸. or CHR⁶-NHR¹¹.

Process variant (vii) initially produces compounds of formula (I) wherein A-B is NR^{11'}-CR⁸R⁹.

Process variant (viii) initially produces compounds of formula (I) wherein A-B is O-CH₂.

Process variant (ix) initially produces compounds where AB is NR¹¹SO₂.

Process variant (x) initially produces compounds of formula (I) wherein one of A and B is CH₂ and the other is NHR¹¹, O or S.

Process variant (xi) initially produces compounds of formula (I) wherein A-B is OCH₂ or CH₂O, providing that if A is CH₂, n = 1.

Process variant (xii) produces compounds where AB is NR¹¹CO.

Process variant (xiii) produces compounds where AB is -CH₂CH₂- or -CH=CH-.

In process variant (v) the reaction is a standard amide formation reaction involving e.g.:
1. Activation of a carboxylic acid (e.g. to an acid chloride, mixed anhydride, active ester, O-acyl-isourea or other species), and treatment with an amine (Ogliaruso, M.A.; Wolfe, J.F. in *The Chemistry of Functional Groups (Ed. Patai, S.) Suppl. B: The Chemistry of Acid Derivatives, Pt. 1* (John Wiley and Sons, 1979), pp 442-8; Beckwith, A.L.J. in *The Chemistry of Functional Groups (Ed. Patai, S.) Suppl. B: The Chemistry of Amides (Ed. Zabricky, J.)* (John Wiley and Sons, 1970), p 73 ff. The acid and amine are preferably reacted in the presence of an activating agent such as 1-(dimethylaminopropyl)-3-ethylcarbodiimide hydrochloride (EDC) or 1-hydroxybenzotriazole (HOBT) or O-(7-azabenzotriazol-1-yl)-*N,N,N',N'*-tetramethyluronium hexafluorophosphate (HATU); or
2. The specific methods of:
   a*. in situ* conversion of an acid into the amine component by a modified Curtius reaction procedure (Shioiri, T., Murata, M., Hamada, Y., *Chem. Pharm. Bull.* 1987, 35, 2698)
   b. *in situ* conversion of the acid component into the acid chloride under neutral conditions (Villeneuve, G. B.; Chan, T. H., *Tetrahedron. Lett.* 1997, 38, 6489).

A' may be, for example protected hydroxymethylene.

In process variant (i) the process is two step: firstly a condensation using a base, preferably sodium hydride or alkoxide, sodamide, alkyl lithium or lithium dialkylamide, preferably in an aprotic solvent e.g. ether, THF or benzene; secondly, hydrolysis using an inorganic acid, preferably HCl in aqueous organic solvent at 0-100°C. Analogous routes are described in DE330945, EP31753, EP53964 and H. Sargent, J. Am. Chem. Soc. **68**, 2688-2692 (1946). Similar Claisen methodology is described in Soszko et. al., Pr.Kom.Mat. Przyr.Poznan.Tow.Przyj.Nauk., (1962), 10, 15.

In process variant (ii) the reaction is carried out in the presence of a base, preferably organometallic or metal hydride e.g. NaH, lithium diisopropylamide or NaOEt, preferably in an aprotic solvent, preferably THF, ether or benzene at -78 to 25°C (analogous process in Gutswiller et al. (1978) J. Am. Chem. Soc. 100, 576).

In process variants (iii) and (iv) if a base is used it is preferably NaH, KH, an alkyl lithium e.g. BuLi, a metal alkoxide e.g. NaOEt, sodamide or lithium dialkylamide e.g.diisopropylamide. An analogous method is described in US 3989691 and M.Gates et. al. (1970) J. Amer.Chem.Soc., 92, 205, as well as Taylor et al. (1972) JACS 94, 6218.

In process variant (vi) the reaction is a standard reductive alkylation using, e.g., sodium borohydride or sodium triacetoxyborohydride (Gribble, G. W. in *Encyclopedia of Reagents for Organic Synthesis (Ed. Paquette, L. A.)* (John Wiley and Sons, 1995), p 4649).

The process variant (vii) is a standard alkylation reaction well known to those skilled in the art, for example where an alcohol or amine is treated with an alkyl halide in the presence of a base (for example see March, J; *Advanced Organic Chemistry, Edition 3* (John Wiley and Sons, 1985), p364-366 and p342-343). The process is preferably carried out in a polar solvent such as N,N-dimethylformamide

In process variant (viii) where X is W such as halogen, methanesulphonyloxy or trifluoromethanesulphonyloxy, the hydroxy group in Y is preferably converted to an OM group where M is an alkali metal by treatment of an alcohol with a base. The base is preferably inorganic such as NaH, lithium diisopropylamide or sodium. Where X is OH, the hydroxy group in Y is activated under Mitsunobu conditions (Fletcher et.al. J Chem Soc. (1995), 623). Alternatively the X=O and Y=CH₂OH groups can be reacted directly by activation with dichlorocarbodiimide (DCC) (Chem. Berichte 1962, 95, 2997 or Angewante Chemie 1963 75, 377).

In process variant (ix) the reaction is conducted in the presence of an organic base such as triethylamine or pyridine such as described by Fuhrman et.al., J. Amer. Chem. Soc.; **67,** 1245, 1945. The X=NR¹¹'SO₂W or Y=SO₂W intermediates can be formed from the requisite amine e.g. by reaction with SO₂Cl₂ analogously to the procedure described by the same authors Fuhrman et.al., J. Amer. Chem. Soc.; **67**, 1245, 1945.

In process variant (x) where one of X and Y contains NHR¹¹ the leaving group W is halogen and the reaction is a standard amine formation reaction such as direct alkylation described in (Malpass, J. R., in *Comprehensive Organic Chemistry,* Vol. 2 (Ed. Sutherland, I. O.), p 4 ff.) or aromatic nucleophilic displacement reactions (see references cited in *Comprehensive Organic Chemistry,* Vol. 6, p 946-947 (reaction index); Smith, D. M. in *Comprehensive Organic Chemistry,* Vol. 4 (Ed. Sammes, P. G.) p 20 ff.). This is analogous to the methods described in GB 1177849.

In process variant (x) where one of X and Y contains OH or SH, this is preferably converted to an OM or SM group where M is an alkali metal by treatment of an alcohol, thiol or thioacetate with a base. The base is preferably inorganic such as NaH, lithium diisopropylamide or sodium, or, for SH, metal alkoxide such as sodium methoxide. The X/Y group containing the thioacetate SCOR^{x} is prepared by treatment of an alcohol or alkyl halide with thioacetic acid or a salt thereof under Mitsunobu conditions. The leaving group V is a halogen. The reaction may be carried out as described in Chapman et.al., J. Chem Soc., (1956),1563, Gilligan et. al., J. Med. Chem., (1992), **35,** 4344, Aloup et. al., J. Med. Chem. (1987), **30**, 24, Gilman et al., J.A.C.S. (1949), **71,** 3667 and Clinton et al., J.A.C.S. (1948), **70,** 491, Barluenga et al., J. Org. Chem. (1987) 52, 5190. Alternatively where X is OH and Y is CH₂V, V is a hydroxy group activated under Mitsunobu conditions (Fletcher et.al. J Chem Soc. (1995), 623).

In process variant (xi) the reaction is as described in den Hertzog et. al., recl.Trav. Chim. Pays-Bas, (1950),**69**, 700.

In process variant (xii) the leaving group W is preferably chloro, bromo or trifluoromethylsulphonyl and the reaction is the palladium catalysed process known as the "Buchwald" reaction (J. Yin and S. L. Buchwald, Org.Lett., 2000, 2, 1101).

In process variant (xiii) coupling of the acetylene compound (V) with the compound (IV) is accomplished using standard Pd-mediated chemistry, for example using Pd(Ph₃P)₂Cl₂ as the catalyst along with the addition of CuI in a mixture of triethylamine and dimethylformamide. . Selective reduction of the intermediate -C≡C- group is carried out either partially to -CH=CH- over a suitable catalyst eg Linlar catalyst, or fully to - CH₂-CH₂- by other means.

Reduction of a carbonyl group A or B to CHOH can be readily accomplished using reducing agents well known to those skilled in the art, e.g. sodium borohydride in aqueous ethanol or lithium aluminium hydride in ethereal solution. This is analogous to methods described in EP53964, US384556 and J. Gutzwiller *et al, J. Amer. Chem. Soc.,* 1978, 100, 576.

The carbonyl group A or B may be reduced to CH₂ by treatment with a reducing agent such as hydrazine in ethylene glycol, at e.g. 130-160°C, in the presence of potassium hydroxide.

Reaction of a carbonyl group A or B with an organometallic reagent yields a group where R⁶ or R⁸ is OH and R⁷ or R⁹ is alkyl.

A hydroxy group on A or B may be oxidised to a carbonyl group by oxidants well known to those skilled in the art, for example, manganese dioxide, pyridinium chlorochromate or pyridinium dichromate.

A hydroxyalkyl A-B group CHR⁷CR⁹OH or CR⁷(OH)CHR⁹ may be dehydrated to give the group CR⁷=CR⁹ by treatment with an acid anhydride such as acetic anhydride.

An amide carbonyl group may be reduced to the corresponding amine using a reducing agent such as lithium aluminium hydride.

A hydroxy group in A or B may be converted to azido by activation and displacement e.g. under Mitsunobu conditions using hydrazoic acid or by treatment with diphenylphosphorylazide and base, and the azido group in turn may be reduced to amino by hydrogenation.

An example of a group Q¹ convertible to NR² R⁴ is NR^{2'}R^{4'} or halogen. Halogen may be displaced by an amine HNR^{2'}R^{4'} by a conventional alkylation.

When Q¹ Q² together form a protected oxo group this may be an acetal such as ethylenedioxy which can subsequently be removed by acid treatment to give a compound of formula (VI): 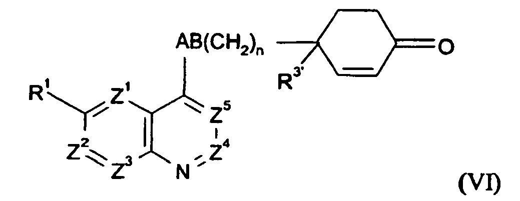 wherein the variables are as described for formula (I)

Intermediates of formula (VI) are novel and as such form part of the invention.

The ketone of formula (VI) is reacted with an amine HNR^{2'}R^{4'} by conventional reductive alkylation as described above for process variant (x).

Other novel intermediates of the invention are compounds of formula (VII): 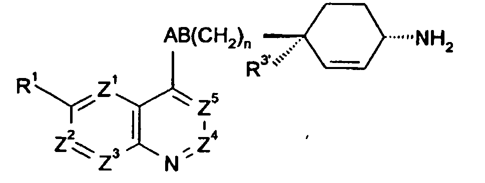 wherein the variables are as described for formula (I).
Examples of groups Z^{1'}, Z^{2'}, Z^{3'}, Z^{4'}, Z^{5'}, are CR^{1a'} where R^{1a'} is a group convertible to R^{1a}. Z^{1'}, Z^{2'}, Z^{3'}, Z^{4'} and Z^{5'} are preferably Z¹, Z², Z³, Z⁴ and Z⁵.

R^{1a'}, R^{1'} and R^{2'} are preferably R^{1a}, R¹ and R². R^{1'} is preferably methoxy. R^{2'} is preferably hydrogen. R^{3'} is R³ or more preferably hydrogen, vinyl, alkoxycarbonyl or carboxy. R^{4'} is R⁴ or more preferably H or an N-protecting group such as t-butoxycarbonyl, benzyloxycarbonyl or 9-fluorenylmethyloxycarbonyl.

Conversions of R^{1a'}, R^{1'}, R^{2'}, R^{3'} and R^{4'} and interconversions of R^{1a}, R¹, R², R³ and R⁴ are conventional. In compounds which contain an optionally substituted hydroxy group, suitable conventional hydroxy protecting groups which may be removed without disrupting the remainder of the molecule include acyl and alkylsilyl groups. N protecting groups are removed by conventional methods.

For example R^{1'} methoxy is convertible to R^{1'} hydroxy by treatment with lithium and diphenylphosphine (general method described in Ireland et. al. (1973) J.Amer.Chem.Soc.,7829) or HBr. Alkylation of the hydroxy group with a suitable alkyl derivative bearing a leaving group such as halide and a protected amino, piperidyl, amidino or guanidino group or group convertible thereto, yields, after conversion/deprotection, R¹ alkoxy substituted by optionally N-substituted amino, piperidyl, guanidino or amidino.

R³ hydroxy may be derivatised by conventional esterification or etherification.

The cyclohexenylamine NH₂ is converted to NR²R⁴ by conventional means such as amide or sulphonamide formation with an acyl derivative for compounds where U or X^{1a} is CO or SO₂ or, where R⁴ is -CH₂R⁵₁ or U or X^{1a} is CH₂, by alkylation with an alkyl halide or other alkyl derivative R₄-W in the presence of base, acylation/reduction or reductive alkylation with an aldehyde.

Where one of R⁶, R⁷, R⁸ or R⁹ contains a carboxy group and they may together with R³ form a cyclic ester linkage. This linkage may form spontaneously during coupling of the compounds of formulae (IV) and (V) or in the presence of standard peptide coupling agents.

It will be appreciated that under certain circumstances interconvertions may interfere, for example, hydroxy groups in A or B and R³ OH and the cyclohexenylamine will require protection e.g. as a carboxy- or silyl-ester group for hydroxy and as an acyl derivative for nitrogen, during conversion of R^{1a'}, R^{1'}, R^{2'}, R^{3'} or R^{4'}, or during the coupling of the compounds of formulae (IV) and (V).

Compounds of formulae (IV) and (V) are known compounds, (see for example Smith *et al, J. Amer. Chem. Soc.,* 1946, 68, 1301) or prepared analogously, see for example the references cited above.

The 4-amino derivatives are commercially available or may be prepared by conventional procedures from a corresponding 4-chloro derivative by treatment with ammonia (O.G. Backeberg et. al., J. Chem Soc., 381, 1942) or propylamine hydrochloride (R. Radinov et. al., Synthesis, 886, 1986).

4-Alkenyl compounds of formula (IV) may be prepared by conventional procedures from a corresponding 4-halogeno-derivative by e.g. a Heck synthesis as described in e.g. *Organic Reactions,* 1982, 27, 345.

4-Halogeno derivatives of compounds of formula (IV) are commercially available, or may be prepared by methods known to those skilled in the art. A 4-chloroquinoline is prepared from the corresponding quinolin-4-one by reaction with phosphorus oxychloride (POCl₃) or phosphorus pentachloride, PCl_{5.} A-4-bromo-substituent may be prepared from the quinolin- or naphthyridin-4-one by reaction with phosphorus tribromide (PBr3) in DMF. A 4-chloroquinazoline is prepared from the corresponding quinazolin-4-one by reaction with phosphorus oxychloride (POCl₃) or phosphorus pentachloride, PCl₅. A quinazolinone and quinazolines may be prepared by standard routes as described by T.A. Williamson in *Heterocyclic Compounds,* 6, 324 (1957) Ed. R.C. Elderfield. Where the required compound of formula (IV) is a 4-halo-5,6-disubstituted quinoline (Z¹=CR^{1a}), the corresponding 5,6-disubstituted quinolin-4-one may be prepared from a 6-bromo-3,4-disubstituted aniline, by condensation with 2,2-dimethyl-[1,3]dioxane-4,6-dione and triethylorthoformate followed by heating of the resulting 2,2-dimethyl-5-[(arylamino)methylidene]-1,3-dioxane-4,6-dione intermediate in refluxing Dowtherm A, to produce the corresponding 8-bromo-5,6-disubstituted quinolin-4-one. Hydrogenolytic removal of the bromine atom using hydrogen under palladium catalysis then generates the required 5,6-disubstituted quinolin-4-one.

Activated carboxy derivatives X=A'COW of formula (IV) may be prepared from X=A'CO₂H derivatives in turn prepared from CO₂H derivatives by conventional methods such as homologation.

4-Carboxy derivatives of compounds of formula (IV) are commercially available or may be prepared by conventional procedures for preparation of carboxy heteroaromatics well known to those skilled in the art. For example, quinazolines may be prepared by standard routes as described by T.A. Williamson in *Heterocyclic Compounds,* 6, 324 (1957) Ed. R.C. Elderfield. These 4-carboxy derivatives may be activated by conventional means, e.g. by conversion to an acyl halide or anhydride.

4-Carboxy derivatives such as esters may be reduced to hydroxymethyl derivatives with for example lithium aluminium hydride. Reaction with mesyl chloride and triethylamine would give the mesylate derivative. A diazo compound (X is -CH=N₂) may be prepared from the 4-carboxaldehyde via the tosyl hydrazone. The 4-carboxaldehyde may be obtained from from the acid by standard procedures well known to those skilled in the art.

A 4-oxirane derivative of compounds of formula (IV) is conveniently prepared from the 4-carboxylic acid by first conversion to the acid chloride with oxalyl chloride and then reaction with trimethylsilyldiazomethane to give the diazoketone derivative. Subsequent reaction with 5M hydrochloric acid gives the chloromethylketone. Reduction with sodium borohydride in aqueous methanol gives the chlorohydrin which undergoes ring closure to afford the epoxide on treatment with base, e.g. potassium hydroxide in ethanol-tetrahydrofuran.

Alternatively and preferably, 4-oxirane derivatives can be prepared from bromomethyl ketones which can be obtained from 4-hydroxy compounds by other routes well known to those skilled in the art. For example, hydroxy compounds can be converted to the corresponding 4-trifluoromethanesulphonates by reaction with trifluoromethanesulphonic anhydride under standard conditions (see K. Ritter, Synthesis, 1993, 735). Conversion into the corresponding butyloxyvinyl ethers can be achieved by a Heck reaction with butyl vinyl ether under palladium catalysis according to the procedure of W. Cabri *et al*, J. Org. Chem, 1992, **57** (5), 1481. (Alternatively, the same intermediates can be attained by Stille coupling of the trifluoromethanesulphonates or the analaogous chloro derivatives with (1-ethoxyvinyl)tributyl tin, T. R. Kelly, J. Org. Chem., 1996, **61**, 4623.) The alkyloxyvinyl ethers are then converted into the corresponding bromomethylketones by treatment with N-bromosuccinimide in aqueous tetrahydrofuran in a similar manner to the procedures of J. F. W. Keana, J. Org. Chem., 1983, **48**, 3621 and T. R. Kelly, J. Org. Chem., 1996, **61**, 4623.

The 4-hydroxyderivatives can be prepared from an aminoaromatic by reaction with methylpropiolate and subsequent cyclisation, analogous to the method described in N. E. Heindel et al, J. Het. Chem., 1969, **6**, 77. For example, 5-amino-2-methoxy pyridine can be converted to 4-hydroxy-6-methoxy-[1,5]naphthyridine using this method.

If a chiral reducing agent such as (+) or (-)-B-chlorodiisopinocamphenylborane ['DIP-chloride'] is substituted for sodium borohydride, the prochiral chloromethylketone is converted into the chiral chlorohydrin with ee values generally 85-95% [see C. Bolm *et al, Chem. Ber.* 125, 1169-1190, (1992)]. Recrystallisation of the chiral epoxide gives material in the mother liquor with enhanced optical purity (typically ee 95%).

The (*R)*-epoxide, when reacted with an amine derivative gives ethanolamine compounds as single diastereomers with (R)-stereochemistry at the benzylic position.

Alternatively, the epoxide may be prepared from the 4-carboxaldehyde by a Wittig approach using trimethylsulfonium iodide [see G.A. Epling and K-Y Lin, *J. Het. Chem*., 1987, 24, 853-857], or by epoxidation of a 4-vinyl derivative.

Pyridazines may be prepared by routes analogous to those described in Comprehensive Heterocyclic Chemistry, Volume 3, Ed A.J. Boulton and A. McKillop and napthyridines may be prepared by routes analogous to those described in Comprehensive Heterocyclic Chemistry, Volume 2, Ed A.J. Boulton and A. McKillop.

4-Hydroxy-1,5-naphthyridines can be prepared from 3-aminopyridine derivatives by reaction with diethyl ethoxymethylene malonate to produce the 4-hydroxy-3-carboxylic acid ester derivative with subsequent hydrolysis to the acid, followed by thermal decarboxylation in quinoline (as for example described for 4-Hydroxy-[1,5]naphthyridine-3-carboxylic acid, J. T. Adams *et al., J.Amer.Chem.Soc.,* 1946, **68**, 1317). A 4-hydroxy-[1,5]naphthyridine can be converted to the 4-chloro derivative by heating in phosphorus oxychloride, or to the 4-methanesulphonyloxy or 4-trifluoromethanesulphonyloxy derivative by reaction with methanesulphonyl chloride or trifluoromethanesulphonic anhydride, respectively, in the presence of an organic base. A 4-amino 1,5-naphthyridine can be obtained from the 4-chloro, 4-methanesulphonyloxy or 4-trifluoromethanesulphonyloxy derivative by reaction with n-propylamine in pyridine.

Similarly, 6-methoxy-1,5-naphthyridine derivatives can be prepared from 3-amino-6-methoxypyridine.

1 ,5-Naphthyridines may be prepared by other methods well known to those skilled in the art (for examples see P.A. Lowe in "Comprehensive Heterocyclic Chemistry" Volume 2, p581-627, Ed A.R. Katritzky and C.W. Rees, Pergamon Press, Oxford, 1984).

The 4-hydroxy and 4-amino-cinnolines may be prepared following methods well known to those skilled in the art [see A.R. Osborn and K. Schofield, *J. Chem. Soc.* 2100 (1955)]. For example, a 2-aminoacetopheneone is diazotised with sodium nitrite and acid to produce the 4-hydroxycinnoline with conversion to chloro and amino derivatives as described for 1,5-naphthyridines.

The compounds of formula (V) are either commercially available or may be prepared by conventional methods.

For compounds of formula (V), where Y is NHR^{11'} suitable amines may be prepared from the corresponding 4-substituted cyclohexenyl acid or alcohol. In a first instance, an N-protected cyclohexenyl amine containing an acid bearing substituent, can undergo a Curtius rearrangement and the intermediate isocyanate can be converted to a carbamate by reaction with an alcohol. Conversion to the amine may be achieved by standard methods well known to those skilled in the art used for amine protecting group removal. For example, an acid substituted N-protected cyclohexenyl amine can undergo a Curtius rearrangement e.g. on treatment with diphenylphosphoryl azide and heating, and the intermediate isocyanate reacts in the presence of 2-trimethylsilylethanol to give the trimethylsilylethylcarbamate (T.L. Capson & C.D. Poulter, *Tetrahedron Lett.,* 1984, 25, 3515). This undergoes cleavage on treatment with tetrabutylammonium fluoride to give the 4-amine substituted N-protected compound of formula (V). Alternatively, an acid group (CH₂)ₙ₋₁CO₂H may be converted to (CH₂)ₙNHR¹¹ by reaction with an activating agent such as isobutyl chloroformate followed by an amine R^{11'}NH₂ and the resulting amide reduced with a reducing agent such as LiAlH₄.

In a second instance, an N-protected cyclohexenyl amine containing an alcohol bearing substituent undergoes a Mitsunobu reaction (for example as reviewed in Mitsunobu, *Synthesis,* (1981), 1), for example with phthalimide in the presence of diethyl azodicarboxylate and triphenylphosphine to give the phthalimidoethyl cyclohexenyl amine. Removal of the phthaloyl group, for example by treatment with methylhydrazine, gives the amine of formula (V).

Compounds of formula (V) where n=1 may be prepared from the compound where n=0 by homologation eg starting from a compound of formula (V) where Y=CO₂H.

Compounds of formula (V) with a -C≡CH group may be prepared from the ketone treated with trimethylsilylacetylene and n-butyl lithium in dimethylformamide at low temperature followed by removal of the trimethylsilyl group with potassium carbonate in methanol or a fluoride source such as KF or tetrabutylammonium fluoride.

Compounds of fonnula (V) with a -CONHR¹¹ group may be prepared from the corresponding nitrile by partial hydrolysis under basic conditions.

Compounds of formula (V) substituted by R³ = OH may be prepared from a 1-keto derivative via a cyanohydrin reaction with sodium cyanide/hydrochloric acid in an ether/water two phase system (J. Marco *et al* Tetrahedron, 1999, **55,** (24), 7625), or using trimethylsilylcyanide and zinc iodide catalysis in dichloromethane (A. Abad et al, J. Chem. Soc., Perkin 1, 1996, **17**, 2193), followed by hydrolysis to give the α-hydroxy acid (Compound(V), Y=CO₂H, n=0, R^{3'} =OH and Q¹ is NR^{2'}R^{4'}) or partial hydrolysis to the carboxamide -CONH₂ as described above. In examples where there is trimethylsilyl protection of the alcohol, this is removed under the hydrolysis conditions. It will be appreciated that the amine protecting group eg N-carboxylic acid *tert*-butyl ester may be concommitantly removed during the hydrolysis step, necessitating a standard reprotection with di-*tert*-butyl dicarbonate, giving key intermediates (V) such as (4-carbamoyl-4-hydroxy-cyclohexyl)-carbamic acid *tert*-butyl ester. It is noteworthy that during the cyanohydrin formation there is little or no stereoselectivity with regard to relative stereochemistry, and the (4-carbamoyl-4-hydroxy-cyclohexyl)-carbamic acid *tert*-butyl ester produced in this process is a mixture of *cis* and *trans* stereoisomers.

A Reformatsky reaction with the keto-derivative and an α-bromocarboxylic acid ester and zinc, followed by acid hydrolysis would afford the β-hydroxycarboxylic acid directly (Compound (V) Y=CO₂H, n=1, R^{3'} =OH )..

Compounds of formula (V) substituted by R³ = OH and n=0 may preferably be prepared with control of relative stereochemistry by cycloaddition chemistry. A Diels Alder reaction between acrylamide and acetoxy butadiene gives (1). Elimination of acetic acid and hetero Diels Alder reaction with an *in-situ* generated acyl nitroso compound gives the tricyclic hydroxylamine product (3). The NO bond is cleaved, for example by molybdenum hexacarbonyl or by other methods known in the literature. 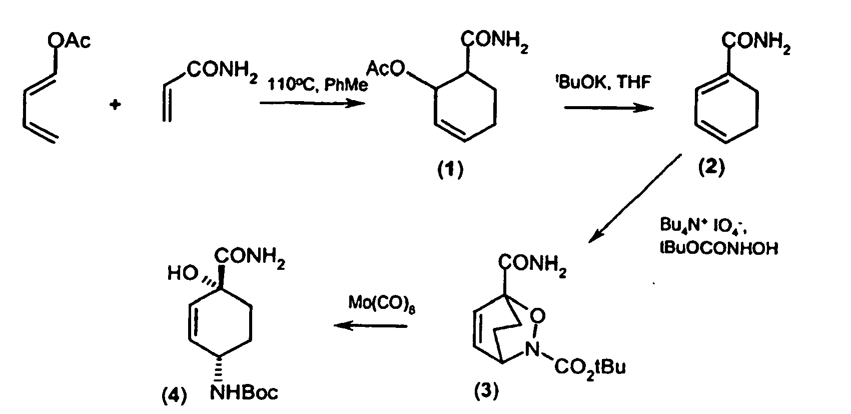 Alternatively an ester of acrylic acid can be used which can subsequently be deprotected and converted to the amide by standard procedures: 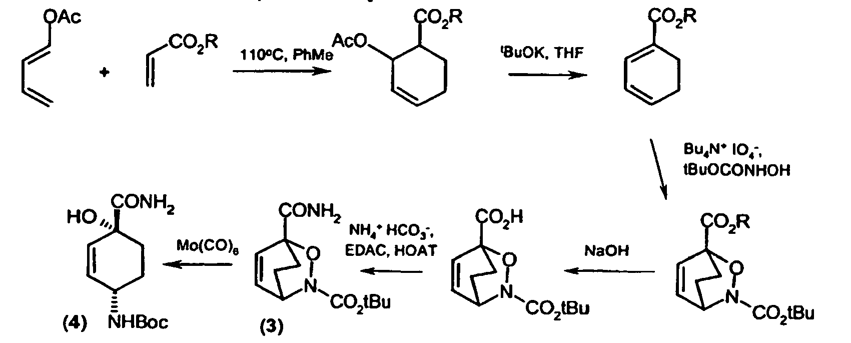 The Boc group in the acyl nitroso component may be replaced by another protecting group which may be subsequently removed during the synthesis and replaced by Boc. The nitroso acyl group and/or the acylate ester moiety may be homochiral allowing the synthesis of individual enantiomers.

R⁴-halides and R⁴-W derivatives, acyl derivatives or aldehydes are commercially available or are prepared conventionally. The aldehydes may be prepared by partial reduction of the corresponding ester with lithium aluminium hydride or diisobutylaluminium hydride or more preferably by reduction to the alcohol, with lithium aluminium hydride or sodium borohydride (see *Reductions by the Alumino- and Borohydrides in Organic Synthesis,* 2nd ed., Wiley, N.Y., 1997; *JOC,* 3197, 1984; *Org. Synth. Coll.,* 102, 1990; 136, 1998; *JOC*, 4260, 1990; *TL,* 995, 1988; *JOC,* 1721, 1999; *Liebigs Ann.lRecl.,* 2385, 1997; *JOC*, 5486, 1987) , followed by oxidation to the aldehyde with manganese (II) dioxide. The aldehydes may also be prepared from carboxylic acids in two stages by conversion to a mixed anhydride for example by reaction with isobutyl chloroformate followed by reduction with sodium borohydride (R. J. Alabaster et al., Synthesis, 598, 1989) to give the hydroxymethyl substituted heteroaromatic or aromatic and then oxidation with a standard oxidising agent such as pyridinium dichromate or manganese (II) dioxide. Acyl derivatives may be prepared by activation of the corresponding ester. R⁴-halides such as bromides may be prepared from the alcohol R⁴OH by reaction with phosphorus tribromide in dichloromethane/triethylamine. Where X^{2a} is CO and X^{3a} is NR^{13a} the R⁴-halide may be prepared by coupling an X^{4a}-NH₂ amine and bromoacetyl bromide. R⁴-W derivatives such as methanesulphonyl derivatives may be prepared from the alcohol R⁴OH by reaction with methane sulphonyl chloride. The leaving group W may be converted to another leaving group W, e.g. a halogen group, by conventional methods. Alternatively the aldehyde R⁵₂CHO and sulphonic acid derivative R⁵₂SO₂W may be generated by treatment of the R⁵₂H heterocycle with suitable reagents. For example benzoxazinones, or more preferably their N-methylated derivatives can be formylated with hexamine in either trifluoroacetic acid or methanesulfonic acid, in a modified Duff procedure [O. I. Petrov et al. *Collect. Czech. Chem. Commun.* **62,** 494-497 (1997)]. 4-Methyl-4H-benzo[1,4]oxazin-3-one may also be formylated using dichloromethyl methyl ether and aluminium chloride giving exclusively the 6-formyl derivative.

Reaction of a R⁵₂H heterocycle with chlorosulphonic acid gives the sulphonic acid derivative (by methods analogous to Techer *et. al., C.R.Hebd. Seances Acad. Sci. Ser.C;* **270**, 1601, 1970).

The aldehyde R⁵₂CHO may be generated by conversion of an R⁵₂halogen or R⁵₂trifluoromethane sulphonyloxy derivative into an olefin with subsequent oxidative cleavage by standard methods. For example, reaction of a bromo derivative under palladium catalysis with trans-2-phenylboronic acid under palladium catalysis affords a styrene derivative which upon ozonolysis affords the required R⁵₂CHO (Stephenson, G. R., Adv. Asymmetric Synth. (1996), 275-298. Publisher: Chapman & Hall, London).

Where R⁵₂ is an optionally substituted benzoimidazol-2-yl group, the compound of formula (V) where R^{4'} is R⁴ may be obtained by converting a R^{4'} cyanomethyl group with partial hydrolysis to give the 2-ethoxycarbonimidoylethyl group which can then be condensed with an appropriately substituted 1,2-diaminobenzene to give the required benzoimidazol-2-yl group.

R⁵₂H heterocycles are commercially available or may be prepared by conventional methods. For example where a benzoxazinone is required, a nitrophenol may be alkylated with for example ethyl bromoacetate and the resulting nitro ester reduced with Fe in acetic acid (alternatively Zn/AcOH/HCl or H₂ Pd/C or H₂ Raney Ni). The resulting amine will undergo spontaneous cyclisation to the required benzoxazinone. Alternatively a nitrophenol may be reduced to the aminophenol, which is reacted with chloroacetyl chloride [method ofX. Huang and C. Chan, *Synthesis* 851 (1994)] or ethyl bromoacetate in DMSO [method of Z. Moussavi et al. *Eur. J. Med. Chim. Ther.* **24**, 55-60 (1989)]. The same general routes can be applied to prepare benzothiazinones [See for example F. Eiden and F. Meinel, Arch. Pharm. 312, 302-312 (1979), H. Fenner and R Grauert *Liebigs. Ann. Chem.* 193-313 (1978)]]. A variety of routes are available to prepare aza analogues of benzothiazinones via the key corresponding aldehydes. For instance, 2-oxo-2,3-dihydro-1*H*-pyrido[3,4-b][1,4]thiazine-7-carbaldehyde may be accessed from 5-fluoro-2-picoline (E. J. Blanz, F. A. French, J. R. DoAmaral and D. A. French, J. Med. Chem. **1970**,13, 1124-1130) by constructing the thiazinone ring onto the pyridyl ring then functionalising the methyl substituent, as described in the Examples. The dioxin analogue of this aza substitution patern, 2,3-dihydro-[1,4]dioxino[2,3-c]pyridine-7-carbaldehyde is accessible from Kojic acid by aminolysis from pyrone to pyridone then annelating the dioxin ring, again as described in the subsequent experimental data. Other aza substitution patterns with pyridothiazin-3-one, pyridooxazin-3-one, and pyridodioxin ring systems are also accessible, again as descibed in the Examples. Ortho-aminothiophenols may be conveniently prepared and reacted as their zinc complexes [see for example V. Taneja et al *Chem. Ind*. 187 (1984)]. Benzoxazolones may be prepared from the corresponding aminophenol by reaction with carbonyl diimidazole, phosgene or triphosgene. Reaction ofbenzoxazolones with diphosporus pentasulfide affords the corresponding 2-thione. Thiazines and oxazines can be prepared by reduction of the corresponding thiazinone or oxazinone with a reducing agent such as lithium aluminium hydride.

The amines R^{2'}R^{4'}NH are available commercially or prepared conventionally. For example amines may be prepared from a bromo derivative by reaction with sodium azide in dimethylformamide (DMF), followed by hydrogenation of the azidomethyl derivative over palladium-carbon. An alternative method is to use potassium phthalimide/DMF to give the phthalimidomethyl derivative, followed by reaction with hydrazine in DCM to liberate the primary amine.

Amines where X^{2a} is CO and X^{3a} is NR ^{13a} may be prepared by reacting an N-protected glycine derivative HO₂C-X^{1a}-NH₂ with X^{4a}-NH₂ by conventional coupling using eg EDC.

Conversions of R^{1a'}, R^{1'}, R^{2'}, R^{3'} and R^{4'} may be carried out on the intermediates of formulae (IV) and (V) prior to their reaction to produce compounds of formula (I) in the same way as described above for conversions after their reaction.

The pharmaceutical compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and crearns.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable salt or *in vivo* hydrolysable ester thereof is administered in the above-mentioned dosage range.

The compound of formula (I) may be the sole therapeutic agent in the compositions of the invention or a combination with other antibiotics or with a β-lactamase inhibitor may be employed.

Compounds of formula (I) are active against a wide range of organisms including both Gram-negative and Gram-positive organisms.

The following examples illustrate the preparation of certain compounds of formula (I) and the activity of certain compounds of formula (I) against various bacterial organisms.

### EXAMPLES

### General Procedure for Reductive Alkylation

The amine (0.32 mmol) under argon in MeOH (10 mL) and DMF (10 mL) was treated with aldehyde (0.32 mmol), and 3A molecular sieves (250 mg), followed by AcOH (1 mL). (Polystyrylmethyl)trimethylammonium cyanoborohydride (0.64 mmol) was added after 2 h. Once the reaction had gone to completion the resin was removed by filtration, and the solvent removed *in vacuo*. The residue was purified by flash column chromatography (silica gel, 0-12% of 2M NH₃/MeOH in dichloromethane) to give the title compound.

### Intermediate 1

### 1,1,1-Trifluoromethanesulfonic acid 6-methoxy-[1,5]naphthyridin-4-yl ester (I1)

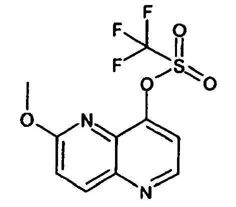

### Method A

### (a) 4-Hydroxy-6-methoxy-[1,5]-naphthyridine

5-Amino-2-methoxypyridine (55 g, 0.44 mol) in methanol (1000 mL) with methyl propiolate (40 mL, 0.44 mol) was stirred for 48 hours, then evaporated and the product purified by chromatography on silica gel (dichloromethane) followed by recrystallisation from dichloromethane-hexane (44.6 g, 48%).
The unsaturated ester (10.5g, 0.05 mol) in warm Dowtherm A (50 mL) was added over 3 minutes to refluxing Dowtherm A, and after a further 20 minutes at reflux the mixture was cooled and poured into diethyl ether. The precipitate was filtered to give a solid (6.26 g, 70%).

### (b) 1,1,1-Trifluoro-methanesulfonic acid 6-methoxy-[1,5]naphthyridin-4-yl ester

4-Hydroxy-6-methoxy-[1,5]-naphthyridine (1a Method A) (10 g, 0.057 mol) in dichloromethane (200 mL) containing 2,6-lutidine (9.94 mL, 0.086 mol) and 4-dimethylaminopyridine (0.07 g, 0.0057 mol) was cooled in ice and treated with trifluoromethanesulfonic anhydride (10.5 mL, 0.063 mol). After stirring for 2.5 h the mixture was washed with saturated ammonium chloride solution, dried, evaporated and purified on silica gel (dichloromethane) to give a solid (13.2 g).

### Method B

### (a) 2,2-Dimethyl-5-({[6-(methoxy)-3-pyridinyl]amino}methylidene)-1,3-dioxane-4,6-dione

A mixture of 6-(methoxy)-3-pyridinamine (50g, 403 mmol), 2,2-dimethyl-1,3-dioxane-4,6-dione (68g, 472 mmol) and triethylorthoformate (66g, 446 mmol) in ethanol (300 ml) was heated to reflux for 3 hours, then left for 2 days at room temperature. Filtration and drying afforded a white solid (102g, 91%). MS (APCI⁺) *m*/*z* 279 (MH⁺).

### (b) 6-(Methoxy)-1,5-naphthyridin-4(1H)-one

Meldrum's adduct (1a Method B) (51g, 183 mmol) was added portionwise over 5 minutes to refluxing Dowtherm A (300ml) (NB Dowtherm A is a commercially available eutectic mixture comprising 26.5% biphenyl and 73.5% biphenyl ether). After the addition was complete, heating was continued for a further 5 minutes then allowed to cool to room temperature. The solution was added to ether (600 ml) and the resulting suspension filtered affording a pale brown solid (24g, 74%).
MS (APCI⁺) *m*/*z* 177 (MH⁺).

### (c) 1,1,1-Trifluoromethanesulfouic acid 6-methoxy-[1,5]naphthyridin-4-yl ester

A suspension of naphthyridone (1b Method B) (25 g, 0.142 mol) in dichloromethane (300 mL) at 0°C was treated, after 30 minutes, with 2,6-lutidine (25 mL, 0.21 mol) and 4-dimethylaminopyridine (1.5g, 12.3 mmol). After a further 15 minutes, a solution of triflic anhydride (26.5 mL, 0.158 mol) in dichloromethane was added dropwise over 30 minutes. The reaction mixture was stirred at 0°C for 45 minutes and at room temperature for a further 30 minutes. The mixture was then washed with saturated ammonium chloride, dried over magnesium sulfate and evaporated in vacuo. The solid residue was purified by flash chromatography on silica gel (eluent dichloromethane) to afford a white solid (24.8 g, 57%).
MS (APCI⁺) *m*/*z* 309 (MH⁺).

### Intermediate 2

### 3-Oxo-3,4-dihydro-2H-pyrido[3,2-b][1,4]thiazine-6-carboxaldehyde (I2)

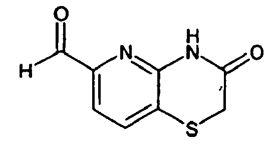

### (a) Methyl 3-oxo-3,4-dihydro-2H-pyrido[3,2-b][1,4]thiazine-6-carboxylate

A solution of ethyl 2-mercaptoacetate (1.473 mL) in DMF (48 mL) was ice-cooled and treated with sodium hydride (540 mg of a 60% dispersion in oil). After 1 hour methyl 6-amino-5-bromopyridine-2-carboxylate (3 g) (T.R. Kelly and F. Lang, *J. Org. Chem. 61,* **1996,** 4623-4633) was added and the mixture stirred for 16 hours at room temperature. The solution was diluted with EtOAc (1 litre), washed with water (3 x 300 mL), dried and evaporated to about 10 mL. The white solid was filtered off and washed with a little EtOAc to afford the title compound (0.95 g).
MS (APCI⁻) *m*/*z* 223 ([M-H]⁻, 100%)

### (b) 3-Oxo-3,4-dibydro-2H-pyrido[3,2-b][1,4]thiazine-6-carboxylic acid

A solution of methyl ester (2a) (788 mg) in dioxan (120 mL)/water (30 mL) was treated dropwise over 2 h with 0.5M NaOH solution (8 mL) and stirred overnight. After evaporation to approx. 3 mL, water (5 mL) was added and 2N HCl to pH4. The precipitated solid was filtered off, washed with a small volume of water and dried under vacuum to give the title compound as a solid (636 mg). MS (APCI⁻) *m*/*z* 209 ([M-H]⁻, 5%), 165([M-COOH]⁻, 100%)

### (c) 6-Hydroxymethyl-3-oxo-3,4-dihydro-2H-pyrido[3,2-b][1,4]thiazine

A solution of carboxylic acid (2b) (500 mg) in THF (24 mL) with triethylamine (0.396 mL) was cooled to -10°C and isobutyl chloroformate (0.339 mL) added. After 20 min the suspension was filtered through kieselguhr into an ice-cooled solution of sodium borohydride (272 mg) in water (8 mL), the mixture stirred 30 min and the pH reduced to 7 with dilute HCl. The solvent was evaporated and the residue triturated under water. The product was filtered and dried under vacuum to give the title compound as a white solid (346 mg).
MS (APCI⁻) *m*/*z* 195 ([M-H]⁻, 50%), 165(100%)

### (d) 3-Oxo-3,4-dihydro-2H-pyrido[3,2-b][1,4]thiazine-6-carboxaldehyde

A solution of alcohol (2c) (330 mg) in dichloromethane (30 mL)/THF (30 mL) was treated with manganese dioxide (730 mg) and stirred at room temperature. Further manganese dioxide was added after 1 h (730 mg) and 16 h (300 mg). After a total of 20 h the mixture was filtered through kieselguhr and the filtrate evaporated. The product was triturated with EtOAc/hexane (1:1) and collected to give the title compound as a solid (180 mg).
MS (APCI⁻) *m*/*z* 195 ([M-H]⁻, 95%), 165 (100%)

### Intermediate 3

### 3-Oxo-3,4-dihydro-2H-pyrido[3,2-b][1,4]oxazine-6-carboxaldebyde (I3)

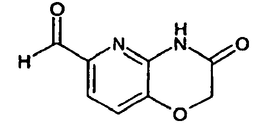

### (a) 2-Bromo-5-hydroxy-6-nitropyridine

3-Hydroxy-2-nitropyridine (20 g, 0.143 mol) was dissolved in methanol (400 mL) and a solution of 25% sodium methoxide in methanol (33 mL, 0.13 mol) was added at room temperature. The mixture was stirred for 30 min, then was cooled to 0 °C, and bromine (7.2 mL, 0.14 mol) was added slowly. The reaction was then stirred at 0 °C for 30 min, then was quenched with glacial AcOH (2.5 mL). The solvent was removed *in vacuo* to afford material (30 g, 96%), which was used without further purification.
MS (ES) *mlz* 219.0 (M + H)⁺.

### (b) Ethyl (6-bromo-2-nitro-pyridin-3-yloxy)acetate

Nitropyridine (3a) (30 g, 0.14 mol) was suspended in acetone (200 mL), and potassium carbonate (39 g, 0.28 mol) was added, followed by ethyl bromoacetate (15.7 ml, 0.14 mol). The reaction was heated at reflux for 10 h, then was cooled to room temperature and diluted with Et₂O. The precipitate was removed by suction filtration, and the filtrate was concentrated *in vacuo* to afford material (38 g, 89%), which was used without further purification.
MS (ES) *m*/*z* 305.0 (M + H)⁺.

### (c) 6-Bromo-4H-pyrido[3,2-b][1,4]oxazin-3-one

Ethyl ester (3b) (38 g, 0.125 mol) was dissolved in glacial AcOH (150 mL), and iron powder (20 g, 0.36 mol) was added. The mixture was mechanically stirred and heated at 90 °C for 5 h, then was cooled to room temperature and diluted with EtOAc (300 mL). The mixture was filtered through a pad of silica gel and the filtrate was concentrated *in vacuo* and the residue recrystallized from MeOH (15 g, 52%).
MS (ES) *m*/*z* 229.0 (M + H)⁺.

### (d) 6-((E)-Styryl)-4H-pyrido[3,2-b][1,4]oxazin-3-one

Pyridooxazinone (3c) (6.0 g, 26.3 mmol) and *trans*-2-phenylvinylboronic acid (3.9 g, 26.3 mmol) were dissolved in 1,4-dioxane (150 mL) and the solution was degassed with argon. (Ph₃P)₄Pd (230 mg, 0.2 mmol) was added, followed by a solution of potassium carbonate (6.9 g, 50 mmol) in H₂O (20 mL). The reaction was heated at reflux under argon overnight, then was cooled to room temperature and diluted with EtOAc (200 mL). The solution was washed sequentially with H₂O and brine, dried (Na₂SO₄), and concentrated *in vacuo.* The solid residue was purified by flash chromatography on silica gel (5-10% EtOAc/CHC]₃) to afford a solid (2.5 g, 38%). MS (ES) *m*/*z* 253.0 (M + H)⁺.

### (e) 3-Oxo-3,4-dihydro-2H-pyrido[3,2-b][1,4]oxazine-6-carboxaldehyde

Pyridooxazinone (3d) (1.2 g, 4.8 mmol) was dissolved in DCM (200 mL) and the solution was cooled to -78 °C. Ozone was bubbled through the solution with stirring until a pale blue colour appeared, then the excess ozone was removed by bubbling oxygen through the solution for 15 min. Dimethylsulfide (1.76 mL, 24 mmol) was added to the solution, and the reaction was stirred at -78 °C for 3 h, then at room temperature overnight. The solvent was removed *in vacuo*, and the residue was triturated with Et₂O (50 mL). The collected solid was washed with additional Et₂O and dried to afford a solid (700 mg, 82%).
MS (ES) *m*/*z* 179.0 (M + H)⁺.

### Intermediate 4

### (1R,4S)-4-Amino-1-hydroxy-cyclohex-2-enecarboxylic acid (6-methoxy-[1,5]naphthyridin-4-yl)-amide and (1S,4R)-4-Amino-1-hydroxy-cyclohex-2-enecarboxylic acid (6-methoxy-[1,5]naphthyridin-4-yl)-amide (I4)

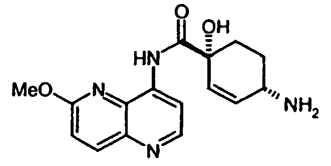

### (a) Acetic acid 6-carbamoyl-cyclohex-2-enyl ester

1-Acetoxy-1,3-butadiene (20.79 g, 185 mmol) was dissolved in toluene (21 mL). To this was added acrylamide (11.98 g, 168 mmol) and hydroquinone (111 mg). The colourless solution was heated at 110°C for 116 h under argon. More 1-acetoxy-1,3-butadiene (5.67 g, 51 mmol) was then added, and heating continued for a further 24 h. The solution was cooled then DCM added. This solution was purified by Biotage 75 chromatography twice on silica gel (2 x 400 g) (DCM:MeOH 0-3%) to give the title compound as a viscous oil (21.76 g, 119 mmol, 70%), which solidified on standing; δH (CDCl₃) 1.83-2.33 (7H, m), 2.51-2.66 (1H, m), 5.54-6.05 (5H, m).

### (b) Cyclohexa-1,3-dienecarboxylic acid amide

To acetic acid ester (4a) (4.00 g, 21.8 mmol) in dry THF (75 mL) was added, over 20 min, potassium *tert*-butoxide in THF (1 M, 24 mL, 24 mmol). After stirring for 2.8 h, EtOAc (300 mL) was added and the solution washed with water (20 mL). The organic phase was dried (MgSO₄), filtered, and concentrated *in vacuo* to give the title compound as a brown oil (>100%). This was used immediately without further purification.

### (c) 1-Carbamoyl-2-oxa-3-aza-bicyclo[2.2.2]oct-5-ene-3-carboxylic acid tert-butyl ester

To the crude amide (4b) (max 21.8 mmol) in DCM (160 mL) was added *N-*hydroxy carbamic acid *tert*-butyl ester (3.05 g, 22.9 mmol). This solution was cooled in an ice bath then a solution of tetrabutylammonium periodate (9.93 g, 22.9 mmol) in DCM (30 mL) was added dropwise. After stirring for a further 17 h the mixture was reduced to a small volume *in vacuo* then diluted with EtOAc. The mixture was then washed with water, aqueous sodium bisulphite (x2), and brine, dried (MgSO₄) and concentrated *in vacuo* to give a residue which was purified by flash column chromatography (silica gel, Pet 40-60:EtOAc 2-60%), to give the title compound as a white solid (1.77 g, 6.96 mmol, 32%).
δH (CDCl₃) 1.47 (9H s), 1.52-1.62(1H, m), 1.74-1.84 (1H, m), 2.12-2.20 (1H, m), 4.73-4.78 (1H, m), 5.48 (1H, br s), 6.57-6.62 (3H, m).
*m*/*z* (ES+) 277 (MNa⁺, 100%).

### (d) (4-Carbamoyl-4-hydroxy-cyclohex-2-enyl)-carbamic acid tert-butyl ester

To *tert*-butyl ester (4c) (998 mg, 3.93 mmol) in MeCN:H₂O (15:1) (70 mL) was added molybdenum hexacarbonyl (2.07 g, 7.85 mmol) and the mixture heated to reflux. After 14 h the reaction mixture was concentrated *in vacuo* and the residue was purified by flash column chromatography (silica gel, DCM:MeOH 0-10%), to give the title compound as a white solid (454 mg, 1.77 mmol, 45%).
δH (CD₃OD) 1.44 (9H s), 1.60-1.71 (1H, m), 1.77-1.82 (1H, m), 1.91-1.98 (1H, m), 2.05-2.12 (1H, m), 4.03-4.07 (1H, m), 5.62-5.65 (1H, m), 5.81-5.84 (1H, m).
*m*/*z* (ES+) 279 (MNa⁺, 100%).

### Enantiomeric resolution of intermediate 4d by chiral HPLC.

Intermediate 4d (0.5g) was dissolved in ethanol (10mL) and applied to a column of ChiralPak AS (100 x 200 mm, 20u) . Elution with 80:20 hexane: isopropyl alcohol was carried out with a flow rate of 450 mL/min, and UV detection at 220 nm. A total of 2g was separated in 4 runs of 0.5 g each, yielding the separate enantiomers:
**E1** (0.856 g) alpha D +111° ( c= 0.7 CH₃OH) with retention time 5.3 mins on analytical chiral HPLC (Chiralpak AS 4.6x 150 mm, 10u, 1.0 mL/min, 80:20 hexane:isopropyl alcohol).
**E2** (0.792g) alpha D -113° ( c= 0.7 CH₃OH) with retention time 7.6 mins on analytical chiral HPLC (Chiralpak AS 4.6x 150 mm, 10u, 1.0 mL/min, 80:20 hexane:isopropyl alcohol).

### (e) [4-Hydroxy-4-(6-methoxy-[1,5]naphthyridin-4-ylcarbamoyl)-cyclohex-2-enyl]-carbamic acid tert-butyl ester

A mixture of ester (4d) (427 mg, 1.67 mmol), cesium carbonate (689 mg, 2.11 mmol), tris(dibenzylideneacetone)dipalladium(0) (30.5 mg, 0.033 mmol), and rac-2,2'-bis(diphenylphosphino)-1,1'-binaphthyl (62 mg, 0.1 mmol) in dry 1,4-dioxane (22 mL) under argon, was sonicated for 20 minutes then 1,1,1-trifluoromethanesulfonic acid 6-methoxy-[1,5]naphthyridin-4-yl ester Intermediate 1 (514 mg, 1.67 mmol) added, and the mixture stirred and heated under argon at 60°C. After 15 h the mixture was cooled, filtered, and the filtrate concentrated *in vacuo* to give a residue which was purified by flash column chromatography (silica gel, DCM:MeOH 0-5%), to give the title compound as a white solid (364 mg, 0.877 mmol, 53%).
δH (CD₃OD) 1.46 (9H s), 1.71-1.81 (1H, m), 1.91-1.97 (1H, m), 2.02-2.07 (1H, m), 2.22-2.29 (1H, m), 4.10-4.16 (1H, m), 4.14 (3H, s), 5.75-5.79 (1H, m), 5.94-5.96 (1H, m), 7.28 (1H, d), 8.21 (1H, d), 8.51 (1H, d), 8.64 (1H, d).
*m*/*z* (ES+) 415 (MH⁺, 100%).

### (f) 4-Amino-1-hydroxy-cyclohex-2-enecarboxylic acid (6-methoxy-[1,5]naphthyridin-4-yl)-amide

[*tert*-butyl ester (4e) (362 mg, 0.873 mmol) in dry DCM (30 mL) was treated with trifluoroacetic acid (10 mL). After 30 min the solvent was removed *in vacuo* and the residue purified by flash column chromatography (silica gel, DCM:MeOH/NH₃ (2M) 0-10%) to give the title compound as a white solid (242 mg, 0.771 mmol, 88%).
δ_{H} (CD₃OD) 1.61-1.70 (1H, m), 1.91-1.98 (1H, m), 2.02-2.08 (1H, m), 2.19-2.27 (1H, m), 3.41-3.45 (1H, m), 4.14 (3H, s), 5.72-5.76 (1H, m), 5,97-6.01 (1H, m), 7.28 (1H, d), 8.20 (1H, d), 8.51 (1H, d), 8.64 (1H, d).
*m*/*z* (ES+) 315 (MH⁺, 100%).

### Intermediate 5

### 2,3-dihydro[1,4]dioxino[2,3-c]pyridine-7-carbaldehyde (I5)

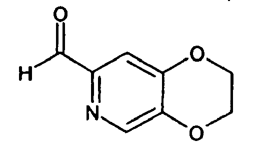

The title compound was prepared as described in Example 24c of WO02056882.

### Intermediate 6

### 2,3-dihydro[1,4]dioxino[2,3-b]pyridine-7-carbaldehyde (I6)

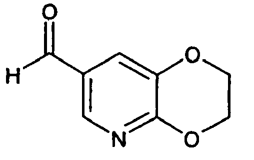

The title compound was prepared as described in Example 40e of WO02056882.

### Intermediate 7

### 2,3-dihydro[1,4]dioxino[2,3-b]pyridine-6-carbaldehyde (I7)

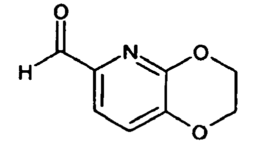

### (a) 6-Methyl-2,3-dihydro-[1,4]dioxino[2,3-b]pyridine-N-oxide

6-Methyl-2,3-dihydro-[1,4]dioxino[2,3-b]pyridine (Example 19b of WO02056882.) (190mg, 1.26mmol) was dissolved in dichloromethane (10mL) and cooled to 0°C. To this solution was added *meta*-chloroperbenzoic acid (388mg, 1.26mmol) and stirring was continued for 5 hours at room temperature. The volatiles were removed under reduced pressure and the residue purified on silica gel using a dichloromethane and methanol gradient. This provided the desired compound as a white solid (146mg, 69%).
MS (APCI+) m/z 168 (MH+).

### (b) Acetic acid 2,3-dihydro-[1,4]dioxino[2,3-b]pyridin-6-ylmethyl ester

N-oxide (7a) (146mg, 0.874mmol) was dissolved in acetic anhydride (5mL). The solution was heated to reflux for 10 hours after which time the volatiles were removed. This afforded the desired product which was used without further purification.

### (c) (2,3-Dihydro-[1,4]dioxino[2,3-b]pyridin-6-yl)-methanol

Ester (7b) (182mg, 0.87mmol) was dissolved in a mixture of tetrahydrofuran and water (1:1, 4mL) and treated with sodium hydroxide (70mg, 1.74mmol). The resulting solution was stirred at room temperature for 12 hours after which time the solvent was removed under reduced pressure. The product obtained in this fashion was used without further purification.

### (d) 2,3-Dihydro-[1,4]dioxino[2,3-b]pyridine-6-carbaldehyde

Alcohol (7c) (145mg, 0.87mmol) was dissolved in dichloromethane (5mL) and treated with manganese dioxide (151mg, 1.74mmol). The resulting slurry was stirred at room temperature and after 5 hours a further batch of manganese dioxide (151mg, 1.74mmol) was added. The slurry was stirred for a further 10 hours and then filtered through Celite and the volatiles removed *in vacuo.* The residue was purified on silica gel to afford the desired product (95mg, 66%).
MS (APCI+) m/z 166 (MH+).

### Intermediate 8

### 6,7-dihydro[1,4]dioxino[2,3-d]pyrimidine-2-carbaldehyde (I8)

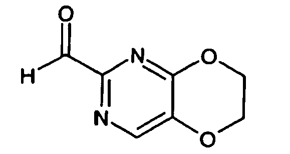

The carboxaldehyde was prepared from 5-benzyloxy-2-hydroxymethyl-3H-pyrimidin-4-one (A. Harris, Aust. J. Chem., 1976, 29, 1335) by hydrogenolysis of the benzyl protecting group and cyclisation with dibromoethane to give (6,7-dihydro-[1,4]dioxino[2,3-d]pyrimidin-2-yl)-methanol followed by oxidation with manganese(II)oxide to afford the product.

### Intermediate 9

### 4-bromo-6-fluoro-5-(methyloxy)quinoline (I9)

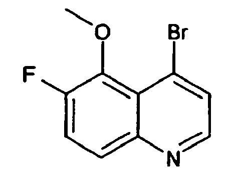

### (a) 4-bromo-2-fluorophenyl ethyl carbonate

A solution of 4-bromo-2-fluorophenol (25 g, 130 mmol) and triethylamine (21.6 mL, 155 mmol) in dichloromethane (120 mL) at 0°C was treated with a solution of ethylcloroformate (14.8 mL, 155 mL) in dichloromaethane (40 mL) added dropwise. The reaction micture was stirred at 0°C for 1 hour and allowed to reach room temperature. The reaction mixture was then washed twice with water. The organic layer was dried over magnesium sulfate and evaporated *in vacuo* to afford the product as a colorless oil (32 g, 93%).
MS (+ve ion electrospray) m/z 263 (MH⁺).

### (b) 4-bromo-2-fluoro-5-nitrophenyl ethyl carbonate

To a solution of (9a) (32 g, 130 mmol) in concentrated sulfuric acid (55mL) at 10°C, fuming nitric acid (8.49 mL, 195 mmol) was added dropwise. After 2 hours, the reaction mixture was poured onto ice/water and extracted several times with ethyl acetate. The combined organic layers were dried over magnesium sulfate and evaporated *in vacuo* to afford the product as a yellow oil (35 g, 93%).
MS (+ve ion electrospray) m/z 309 (MH⁺).

### (c) 4-bromo-2-fluoro-5-nitrophenol

A solution of (9b) (35 g, 113 mmol) in methanol (200 mL) was treated with sodium bicarbonate (19 g, 227 mmol). The reaction mixture was stirred at 60°C for 4 hours. Methanol was evaporated under vacuum. Water (55 mL) was added to the residue and the aqueous layer was acidified to pH5 by addition of a solution of hydrogen chloride 5N. The aqueous layer was extracted twice with ethyl acetate. The combined organic layers were dried over magnesium sulfate and evaporated *in vacuo* to afford the product as a yellow solid (25 g, 93%).
MS (+ve ion electrospray) m/z 237 (MH⁺).

### (d) 4-bromo-2-fluoro-5-nitrophenyl methyl ether

A solution of phenol (9c) (25 g, 106 mmol) in DMF (200 mL) was treated with potassium carbonate (28.9 g, 212 mmol) and methyl iodide (12.8 mL, 212 mmol). The reaction mixture was stirred at 60°C for 5 hours and evaporated in vacuo. The mixture was partitioned between water and ethyl acetate. The organic layer was dried over magnesium sulfate and evaporated in vacuo to afford the product as a yellow solid (25.6 g, 97%).
MS (+ve ion electrospray) m/z 251 (MH⁺).

### (e) 2-bromo-4-fluoro-5-(methyloxy)aniline

A mixture of (9d) (25.5 g, 102 mmol), acetic acid (250 mL), ethanol (250 mL) and iron powder (22.7 g, 408 mmol) was heated at 100°C for 4 hours. The reaction mixture was cooled down to room temperature, diluted with water, neutralised by addition of potassium carbonate and filtered through celite. The aqueous layer was extracted three times with dichloromethane. The combined organic layers were dried over magnesium sulfate and evaporated *in vacuo* to afford the product as a white solid (15 g, 67%).
MS (+ve ion electrospray) m/z 220 (MH⁺).

### (f) 5-({[2-bromo-4-fluoro-5-(methyloxy)phenyl]amino}methylidene)-2,2-dimethyl-1,3-dioxane-4,6-dione

A mixture of (9e) (15 g, 68 mmol), 2,2-dimethyl-[1,3]dioxane-4,6-dione (11.8 g, 82 mmol) and trimethylorthoformate (13.6 ml) in ethanol (70 ml) was refluxed for 3hours. After cooling the solid was filtered off, washed with ethanol and air dried. The product was obtained as an off-white solid (23.3 g, 92%).
MS (+ve ion electrospray) m/z 374 (MH+).

### (g) 8-bromo-6-fluoro-5-(methyloxy)-4(1H)-quinolinone

Intermediate (9f) (13 g, 34.8 mmol) was slowly added over five minutes to refluxing Dowtherm A (40 ml). After an additional five minutes at reflux, the mixture was allowed to cool to room temperature then ether was added. The product was filtered off, thoroughly washed with ether then dried *in vacuo* to afford the product as a gold coloured solid (5.4 g, 57%).
MS (+ve ion electrospray) m/z 273 (MH+).

### (h) 6-fluoro-5-(methyloxy)-4(1H)-quinolinone

A suspension of bromoquinolone (9g) (3.5 g, 12.8 mmol) in dioxan/water 300mL/100mL was treated with a solution of sodium hydroxide 1N (12.8 mL, 12.8 mmol). The solution was hydrogenated with palladium on charcoal. The reaction mixture was filtered through kieselguhr, acidified by addition of a solution of hydrogen bromide and evaporated to dryness. The residue was treated with water (30 mL), filtered and dried in vacuo to afford the product as a white solid (3.0 g, 60%).
MS (+ve ion electrospray) m/z 194 (MH+).

### (i) 4-bromo-5-fluoro-6-(methyloxy)quinoline

To a solution of (9h) (2 g, 10 mmol) in DMF (13 ml) was added dropwise phosphorus tribromide (1.2 ml, 12.4 mmol) over five minutes (slightly exothermic). The reaction was allowed to cool to room temperature and was then diluted with ice water and stirred 1 hour then diluted with additional water. The product was filtered off, washed with water and dried *in vacuo* to afford the product as a white solid (1.8 g, 71%).
MS (+ve ion electrospray) m/z 257 (MH+).

### Intermediate 10

### 4-bromo-6-fluoroquinoline (I10)

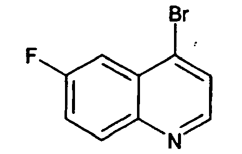

### (a) 5-{[(4-fluorophenyl)amino]methylidene}-2,2-dimethyl-1,3-dioxane-4,6-dione

A mixture of 4-fluoroaniline (21.3 mL, 225 mmol), 2,2-dimethyl-[1,3]dioxane-4,6-dione (38.9 g, 270 mmol) and trimethylorthofonnate (44.9 ml) in ethanol (130 ml) was refluxed for 3hours. After cooling the solid was filtered off, washed with ethanol and air dried. The product was obtained as an off-white solid (55.3 g, 93%).
MS (+ve ion electrospray) m/z 265 (MH+).

### (b) 6-fluoro-4(1H)-quinolinone

Intermediate (10a) (10.9 g, 41 mmol) was slowly added over five minutes to refluxing Dowtherm A (50 ml). After an additional five minutes at reflux, the mixture was allow to cool to room temperature then ether (50 ml) was added. The product was filtered off, thoroughly washed with ether then dried *in vacuo* to afford the product as a gold coloured solid (16.2 g, 94%).
MS (+ve ion electrospray) m/z 164 (MH+).

### (c) 4-bromo-6-fluoroquinoline

To a solution of (10b) (11.3 g, 69.3 mmol) in DMF (350 ml) was added dropwise phosphorous tribromide (7.1 ml, 76.3 mmol) over five minutes (slightly exothermic). The reaction was allowed to cool to room temperature and was then diluted with ice water and stirred 1 hour then diluted with additional water. The product was filtered off, washed with water and dried *in vacuo* to afford the product as a white solid (12.0 g, 76%).
MS (+ve ion electrospray) m/z 226 (MH+).

### Intermediate 11

### 4-bromo-6-fluoro-5-{[2-(methyloxy)ethyl]oxy}quinoline (I11)

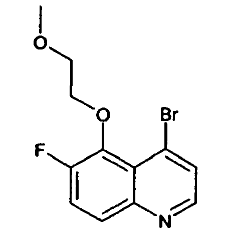

### (a) 1-bromo-5-fluoro-4-{[2-(methyloxy)ethyl]oxy}-2-nitrobenzene

A solution of 4-bromo-2-fluoro-5-nitrophenol (15 g, 59.5 mmol) in DMF (130 mL) was treated with potassium carbonate (16.4 g, 119 mmol) then bromoethyl methyl ether (7 mL, 74.4 mmol). The reaction mixture was stirred at 40°C for 7 hours. DMF was evaporated in vacuo.Partition with water and ethyl acetate. Aqueous layer was extracted several times with ethyl acetate. Combined organic layers were dried over magnesium sulfate and evaporated in vacuo to afford the product as a yellow oil (13 g, 74%).
MS (+ve ion electrospray) m/z 295 (MH+).

### (b) 2-bromo-4-fluoro-5-{[2-(methyloxy)ethyl]oxy}aniline

A mixture of (11a) (11.8 g, 40.2 mmol), acetic acid (120 mL), ethanol (120 mL) and iron powder (9 g, 161 mmol) was heated at 100°C for 4 hours. The reaction mixture was cooled down to room temperature, diluted with water, neutralised by addition of potassium carbonate and filtered through celite. The aqueous layer was extracted three times with dichloromethane. The combined organic layers were dried over magnesium sulfate and evaporated *in vacuo* to afford the product as a white solid (10.2 g, 96%).
MS (+ve ion electrospray) m/z 265 (MH⁺).

### (c) 5-{[(2-bromo-4-fluoro-5-{[2-(methyloxy)ethyl]oxy}phenyl)amino]methylidene}-2,2-dimethyl-1,3-dioxane-4,6-dione

A mixture of (11b) (10.2 g, 38.6 mmol), 2,2-dimethyl-[1,3]dioxane-4,6-dione (6.7 g, 46.3 mmol) and trimethylorthoformate (7.7 ml) in ethanol (45 ml) was refluxed for 3hours. After cooling the solid was filtered off, washed with ethanol and air dried. The product was obtained as an off-white solid (12.9 g, 80%).
MS (+ve ion electrospray) m/z 419 (MH+).

### (d) 8-bromo-6-fluoro-5-{[2-(methyloxy)ethyl]oxy}-4(1H)-quinolinone

Intermediate (11c) (12.9 g, 31 mmol) was slowly added over five minutes to refluxing Dowtherm A (50 ml). After an additional five minutes at reflux, the mixture was allow to cool to room temperature then ether was added. The product was filtered off, thoroughly washed with ether then dried *in vacuo* to afford the product as a gold coloured solid (7.9 g, 81 %).
MS (+ve ion electrospray) m/z 317 (MH+).

### (e) 6-fluoro-5-{[2-(methyloxy)ethyl]oxy}-4(1H)-quinolinone

A suspension of bromoquinolone (11d) (8.2 g, 26 mmol) in dioxan/water 200mL/100mL was treated with a solution of sodium hydroxide 2N (26 mL, 52 mmol). The solution was hydrogenated with palladium on charcoal. The reaction mixture was filtered trough celite, acidified by addition of a solution of hydrogen bromide and evaporated to dryness. The residue was treated with water (30 mL), filtered and dried in vacuo to afford the product as a white solid (7.9 g, 100%).
MS (+ve ion electrospray) m/z 238 (MH+).

### (f) 4-bromo-6-fluoro-5-{[2-(methyloxy)ethyljoxy}quinoline

To a solution of (11e) (3.99 g, 13 mmol) in DMF (35 ml) was added dropwise phosphorus tribromide (1.4 ml, 15.6 mmol) over five minutes (slightly exothermic). The reaction was allowed to cool to room temperature and was then diluted with ice water and stirred 1 hour then diluted with additional water. The product was filtered off, washed with water and dried *in vacuo* to afford the product as a white solid (1.3 g, 33%).
MS (+ve ion electrospray) m/z 301 (MH+).

### Intermediate 12

### 4-bromo-5-fluoro-6-(methyloxy)quinoline (I12)

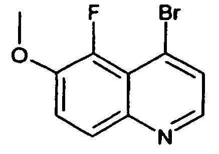

### (a) 2-bromo-5-fluoro-4-(methyloxy)aniline

A solution of bromine (3.15 mL, 61.3 mol) in dichloromethane (100mL) was added dropwise to a suspension of 3-fluoro-4-methoxyaniline (8.65 g, 61.3 mmol) and potassium carbonate (8.9 g, 64.4 mmol) in dichloromethane (200 mL) at -15°C. At the end of the addition, the reaction mixture was stirred for 30 minutes at -15°C. The reaction mixture was then treated with water (250 mL). The organic layer was separated and the aqueous layer was extracted again with dichloromethane. The combined organic layers were washed with a solution of sodium metabisulfite and dried over magnesium sulfate. Evaporation and flash silica chromatography eluting with petroleum ether/ethyl acetate 9/1 afforded the product as a pale yellow solid (10.2 g, 76%).
MS (+ve ion electrospray) m/z 220 (MH+).

### (b) 5-({(2-bromo-5-fluoro-4-(methyloxy)phenyl]amino}methylidene)-2,2-dimethyl-1,3-dioxane-4,6-dione

A mixture of (12a) (10.2 g, 46.5 mmol), 2,2-dimethyl-[1,3]dioxane-4,6-dione (8.0 g, 55.9 mmol) and trimethyl orthoformate (9.3 ml) in ethanol (60 ml) was refluxed for 3hours. After cooling the solid was filtered off, washed with ethanol and air dried. The product was obtained as an off-white solid (15.6 g, 90%).
MS (+ve ion electrospray) m/z 374 (MH+).

### (c) 8-bromo-5-fluoro-6-(methyloxy)-4(1H)-quinolinone

Intermediate (12b) (15.6 g, 41.8 mmol) was slowly added over five minutes to refluxing Dowtherm A (50 ml). After an additional five minutes at reflux, the mixture was allow to cool to room temperature then ether (50 ml) was added. The product was filtered off, thoroughly washed with ethyl acetate (20 mL) and ether (3 x 20 mL) then dried *in vacuo* to afford the product as a gold coloured solid (16.2 g, 94%).
MS (+ve ion electrospray) m/z 273 (MH+).

### (d) 5-fluoro-6-(methyloxy)-4(1H)-quinolinone

A suspension ofbromoquinolone (12c) (3.3 g, 12.3 mmol) in dioxan/water 300mL/100mL was treated with a solution of sodium hydroxide 2N (12.6 mL, 24.6 mmol). The solution was hydrogenated with palladium on charcoal. The reaction mixture was filtered through celite, acidified by addition of a solution of hydrogen bromide and evaporated to dryness. The residue was treated with water (30 mL), filtered and dried in vacuo to afford the product as a white solid (5.2 g, 100%).
MS (+ve ion electrospray) m/z 194 (MH+).

### (e) 4-bromo-5-fluoro-6-(methyloxy)quinoline

To a solution of (12d) (5.2 g, 26.9 mmol) in DMF (150 ml) was added dropwise phosphorus tribromide (2.8 ml, 29.6 mmol) over five minutes (slightly exothermic). The reaction was allowed to cool to room temperature and was then diluted with ice water and stirred 1 hour then diluted with additional water. The product was filtered off, washed with water and dried *in vacuo* to afford the product as a white solid (4.7 g, 69%).
MS (+ve ion electrospray) m/z 256 (MH+).

### Example 1

### (1R,4S)-1-Hydroxy-4-[(3-oxo-3,4-dihydro-2H-pyrido[3,2-b][1,4]thiazin-6-ylmethyl)-amino]-cyclohex-2-enecarboxylic acid (6-methoxy-[1,5]naphthyridin-4-yl)-amide dihydrochloride and (1S,4R)-1-Hydroxy-4-[(3-oxo-3,4-dihydro-2H-pyrido[3,2-b][1,4]thiazin-6-ylmethyl)-amino]-cyclohex-2-enecarboxylic acid (6-methoxy-[1,5]naphthyridin-4-yl)-amide dihydrochloride

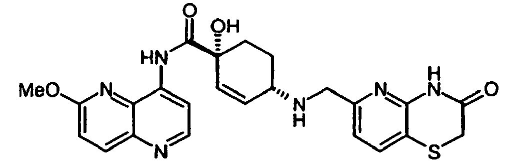

Prepared by the General Procedure for Reductive Alkylation from Intermediate 2 and racemic Intermediate 4f to give the free base of the title compound in 77% yield; δ_{H} (CDCl₃/CD₃OD) 1.68-1.79 (1H, m), 1.95-2.02 (1H, m), 2.07-2.13 (1H, m), 2.19-2.26 (1H, m), 3.32-3.38 (1 H, m), 3.51 (2H, s), 3.91 (2H, s), 4.14 (3H, s), 5.80-5.83 (1H, m), 6.09-6.11 (1H, m), 7.04 (1H, d), 7.25 (1H, d), 7.67 (1H, d), 8.18 (1H, d), 8.50 (1H, d), 8.62 (1H, d); *m*/*z* (ES+) 493 (MH⁺, 100%).

This material as a solution in DCM/MeOH 1:1 was treated with 4M HCl in 1,4-dioxane (0.5 mL), evaporated to dryness, then dried under vacuum to provide the title compound as a white solid.

### Example 2

### (1R,4S)-1-Hydroxy-4-[(3-oxo-3,4-dihydro-2H-pyrido[3,2-b][1,4]oxazin-6-ylmethyl)-amino]-cyclohex-2-enecarboxylic acid (6-methoxy-[1,5]naphthyridin-4-yl)-amide dihydrochloride and (1S,4R)-1-Hydroxy-4-[(3-oxo-3,4-dihydro-2H-pyrido[3,2-b][1,4]oxazin-6-ylmethyl)-amino]-cyclohex-2-enecarboxylic acid (6-methoxy-[1,5]naphthyridin4-yl)-amide dihydrochloride

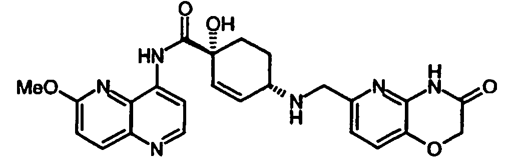

Prepared by the General Procedure for Reductive Alkylation from Intermediate 3 and Intermediate 4f on a 0.305 mmol scale to give the free base of the title compound in 72% yield; δ_{H} (CDCl₃/CD₃OD) 1.67-1.77 (1H, m), 1.98-2.02 (1H, m), 2.08-2.13 (1H, m), 2.21-2.28 (1H, m), 3.34-3.38 (1H, m), 3.88 (2H, s), 4.15 (3H, s), 4.62 (2H, s), 5.81-5.85 (1H, m), 6.07-6.09 (1H, m), 6.96 (1H, d), 7.23 (1H, d), 7.26 (1H, d), 8.19 (1H, d), 8.52 (1H, d), 8.63 (1H, d); *m*/*z* (ES+) 477 (MH⁺, 100%).

This material as a solution in DCM/MeOH 1:1 was treated with 4M HCl in 1,4-dioxane (0.5 mL), evaporated to dryness, then dried under vacuum to provide the title compound as a white solid.

### General synthetic route

The coupling of the corresponding aryl derivative and either the racemic cyclohexenyl amide ( I4d ) or the optically active form (E1 or E2), was carried out as described in the preparation of (4e). After deprotection of the t-butoxycarbonyl group as described in the preparation of compound I4f, the reductive alkylation with the appropriate aldehyde was carried out as described in the General Procedure, with yields as given in the following tables. The compounds were converted to the corresponding dihydrochlorides as described in Example 1.

**Table 1 Methoxynaphthyridines**

| | | | | | | |
|---|---|---|---|---|---|---|
| 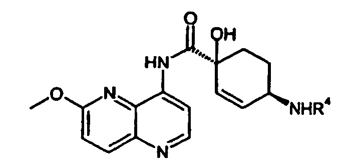 | | | | | | |

| Example | R⁴ | amide | Aryl derivative | Aldehyde | Yield of final product | MS of final product |
|---|---|---|---|---|---|---|
| 3 | 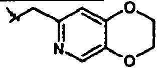 | E1 | I1 | 15 | 74% | ESI 464 (MH⁺, 100%) |
| 4 | 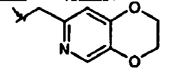 | E2 | I1 | 15 | 43% | ESI 464 (MH⁺, 100%) |
| 5 | 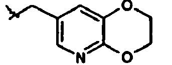 | E2 | I1 | I6 | 43% | ESI464 (MH⁺, 100%) |
| 6 | 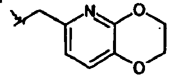 | E2 | I1 | I7 | 7% | ESI464 (MH⁺, 100%) |
| 7 | 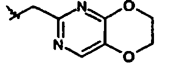 | E2 | I1 | I8 | 25% | ESI 465 (MH⁺, 100%) |

**Table 2 6-Fluoro-5-methoxy Quinolines**

| | | | | | | |
|---|---|---|---|---|---|---|
| 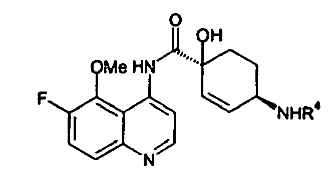 | | | | | | |

| Example | R⁴ | Amide | Aryl derivative | Aldehyde | Yield of final product | M+ of final product |
|---|---|---|---|---|---|---|
| 8 | 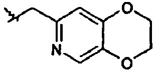 | E1 | I9 | I5 | 56% | ES⁺ 481 (MH⁺,100%) |
| 9 | 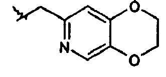 | E2 | I9 | I5 | 62% | ES⁺ 481 (MH⁺, 100%) |

**Table 3 Dioxinopyridines**

| | | | | | | |
|---|---|---|---|---|---|---|
| 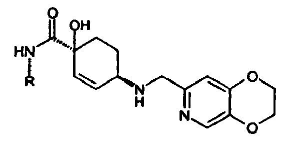 | | | | | | |

| Example | R | Amide | Aryl derivative | Aldehyde | Yield of final product | M+ of final product |
|---|---|---|---|---|---|---|
| 10 | 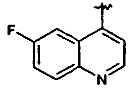 | E2 | I10 | 15 | 12% | ES⁺ 525 (MH⁺, 100%) |
| 11 | 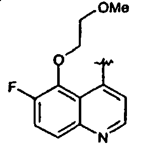 | E2 | I11 | 15 | 34% | ES⁺ 525 (MH⁺, 100%) |
| 12 | 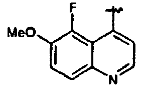 | E2 | I12 | 15 | 40% | ES⁺ 481 (MH⁺, 100%) |

### Biological Activity

The MIC (µg/ml) of test compounds against various organisms was determined including: **S. epidermidis CL7, S. aureus WCUH29, S. pneumoniae 1629, S. pyogenes CN10, H. influenzae ATCC 49247, E.faecalis 2, M. catarrhalis Ravasio, E. coli 7623.**

Examples 1, 2, 3, 4, 5 and 9 have an MIC ≤2 µg/ml versus all these organisms.
Examples 8, 10 have an MIC ≤16 µg/ml versus all these organisms.
Examples 6, 7, 11, 12 have an MIC≤1 µg/ml versus some of these organisms.

## Claims

1. A compound of formula (I) or a pharmaceutically acceptable derivative thereof: 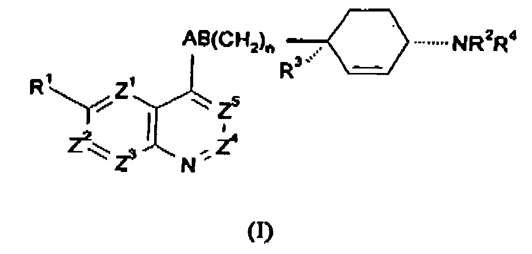 wherein:
one of Z¹, Z², Z³, Z⁴ and Z⁵ is N, one is CR^{1a} and the remainder are CH, or one of Z¹, Z², Z³, Z⁴ and Z⁵ is CR^{1a} and the remainder are CH;
R¹ and R^{1a} are independently selected from hydrogen; hydroxy; (C₁₋₆) alkoxy optionally substituted by (C ₁₋₆)alkoxy, amino, piperidyl, guanidino or amidino any of which is optionally N-substituted by one or two (C ₁₋₆)alkyl, acyl or (C₁₋₆)alkylsulphonyl groups, CONH₂, hydroxy, (C₁₋₆)alkylthio, heterocyclylthio, heterocyclyloxy, arylthio, aryloxy, acylthio, acyloxy or (C₁₋₆)alkylsulphonyloxy; (C ₁₋₆)alkoxy-substituted (C ₁₋₆)alkyl; halogen; (C ₁₋₆)alkyl; (C ₁₋₆)alkylthio; trifluromethyl; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆₎alkylsulphonyl groups, or when Z¹ is CR^{1a}, R¹ and R^{1a} may together represent (C₁₋₂)alkylenedioxy, or when Z⁵ is CR^{1a}, R^{1a} may instead be, cyano, hydroxymethyl or carboxy,
provided that when Z¹, Z², Z³, Z⁴ and Z⁵ are CR^{1a} or CH, then R¹ is not hydrogen;
R² is hydrogen, or (C₁₋₄)alkyl or (C₂₋₄)alkenyl optionally substituted with 1 to 3 groups selected from:
amino optionally substituted by one or two (C₁₋₄)alkyl groups; carboxy; (C₁₋₄)alkoxycarbonyl; (C₁₋₄)alkylcarbonyl; (C₂₋₄)alkenyloxycarbonyl; (C₂₋₄)alkenylcarbonyl; aminocarbonyl wherein the amino group is optionally substituted by hydroxy, (C₁₋₄)alkyl, hydroxy(C₁₋₄)alkyl, aminocarbonyl(C₁₋₄)alkyl, (C₂₋₄)alkenyl, (C₁₋₄)alkylsulphonyl, trifluorornethylsulphonyl, (C₂₋₄)alkenylsulphonyl, (C₁₋₄)alkoxycarbonyl, (C₁₋₄)alkylcarbonyl, (C₂₋₄)alkenyloxycarbonyl or (C_{2- 4})alkenylcarbonyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triaxol-5-yl optionally substituted by R¹⁰; 5-oxo-1,2,4-oxadiazol-3-yl; halogen; (C₁₋₄)alkylthio; trifluoromethyl; hydroxy optionally substituted by (C₁₋₄)alkyl, (C₂₋₄)alkenyl, (C₁₋₄)alkoxycarbonyl, (C₁₋₄)alkylcarbonyl, (C₂₋₄)alkenyloxycarbonyl, (C₂₋₄)alkenylcarbonyl; oxo; (C₁₋₄)alkylsulphonyl; (C₂₋₄)alkenylsulphonyl; or (C₁₋₄)aminosulphonyl wherein the amino group is optionally substituted by (C₁₋₄)alkyl or (C₂₋₄)alkenyl;
R³ is hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl;
R¹⁰ is selected from (C₁₋₄)alkyl and (C₂₋₄)alkenyl either of which may be optionally substituted by a group R¹² as defined above; carboxy; aminocarbonyl wherein the amino group is optionally substituted by hydroxy, (C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl or (C₂₋₆)alkenyl; (C₁₋₆)alkylsulphonyl; trifluoromethylsulphonyl; (C₂₋₆)alkenylsulphonyl; (C₁₋₆)alkoxycarbonyl; (C₁₋₆)alkylcarbonyl; (C₂₋₆)alkenyloxycarbonyl; and (C₂₋₆)alkenylcarbonyl;
R⁴ is a group -CH₂-R⁵₁ in which R⁵₁ is selected from:
(C₄₋₈)alkyl; hydroxy(C₄₋₈)alkyl; (C₁₋₄)alkoxy(C₄₋₈)alkyl; (C₁₋₄)alkanoyloxy(C₄₋₈)alkyl; (C₃₋₈)cycloalkyl(C₄₋₈)alkyl; hydroxy-, (C₁₋₆)alkoxy- or (C₁₋₆)alkanoyloxy-(C₃₋₈)cycloalkyl(C₄₋₈)alkyl; cyano(C₄₋₈)alkyl; (C₄₋₈)alkenyl; (C₄₋₈)alkynyl; tetrahydrofuryl; mono- or di-( C₁₋₆)alkylamino(C₄₋₈)alkyl; acylamino(C₄₋₈)alkyl; (C₁₋₆)alkyl- or acyl-aminocarbonyl(C₄₋₈)alkyl; mono- or di-(C₁₋₆)alkylamino(hydroxy) (C₄₋₈)alkyl; or
R⁴ is a group -U-R⁵₂ where R⁵₂ is an optionally substituted bicyclic carbocyclic or heterocyclic ring system (A): 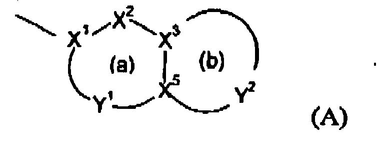 containing up to four heteroatoms in each ring in which
at least one of rings (a) and (b) is aromatic;
X¹ is C or N when part of an aromatic ring or CR¹⁴ when part of a non aromatic ring;
X² is N, NR¹³, O, S(O)ₓ, CO or CR¹⁴ when part of an aromatic or non-aromatic ring or may in addition be CR¹⁴R¹⁵ when part of a non aromatic ring;
X³ and X⁵ are independently N or C;
Y¹ is a 0 to 4 atom linker group each atom of which is independently selected from N, NR¹³, O, S(O)ₓ, CO and CR¹⁴ when part of an aromatic or non-aromatic ring or may additionally be CR¹⁴R¹⁵ when part of a non aromatic ring,
Y² is a 2 to 6 atom linker group, each atom of Y² being independently selected from N, NR¹³, O, S(O)ₓ, CO and CR¹⁴ when part of an aromatic or non-aromatic ring or may additionally be CR¹⁴R¹⁵ when part of a non aromatic ring; each of R¹⁴ and R¹⁵ is independently selected from: H; (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C ₁₋₄)alkylcarbonyloxy; (C₁₋₄)alkoxycarbonyl(C₁₋₄)alkyl; hydroxy; hydroxy(C ₁₋₄)alkyl; mercapto(C ₁₋₄)alkyl; (C ₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; aryl; aryl(C ₁₋₄)alkyl; aryl(C ₁₋₄)alkoxy;
each R¹³ is independently H; trifluoromethyl; (C₁₋₄)alkyl optionally substituted by hydroxy, carboxy, (C₁₋₆)alkoxycarbonyl, (C₁₋₆)alkoxy, (C₁₋₆)alkylthio, halo or trifluoromethyl; (C₂₋₄)alkenyl; aryl; aryl (C₁₋₄)alkyl; arylcarbonyl; heteroarylcarbonyl; (C ₁₋₄)alkoxycarbonyl; (C ₁₋₄)alkylcarbonyl; formyl; (C ₁₋₆)alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl;
each x is independently 0, 1 or 2;
U is CO, SO₂ or CH₂; or
R⁴ is a group -X^{1a}-X^{2a}-X^{3a}-X^{4a} in which:
X^{1a} is CH₂, CO or SO₂;
X^{2a} is CR^{14a}R^{15a};
X^{3a} is NR^{13a}, O, S, SO₂ or CR^{14a}R^{15a}; wherein:
each of R^{14a} and R^{15a} is independently selected from the groups listed above for R¹⁴ and R¹⁵, provided that R^{14a} and R^{15a} on the same carbon atom are not both selected from optionally substituted hydroxy and optionally substituted amino; or
R^{14a} and R^{15a} together represent oxo;
R^{13a} is hydrogen; trifluoromethyl; (C₁₋₆)alkyl; (C₂₋₆)alkenyl; (C₁₋₆)alkoxycarbonyl; (C₁₋₆)alkylcarbonyl; or aminocarbonyl wherein the amino group is optionally substituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl; or
two R^{14a} groups or an R^{13a} and an R^{14a} group on adjacent atoms together represent a bond and the remaining R^{13a}, R^{14a} and R^{15a} groups are as above defined; or
two R^{14a} groups and two R^{15a} groups on adjacent atoms together represent bonds such that X^{2a} and X^{3a} is triple bonded;
X^{4a} is phenyl or C or N linked monocyclic aromatic 5- or 6-membered heterocycle containing up to four heteroatoms selected from O, S and N and: optionally C-substituted by up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; aryl, aryl(C₁₋₄)alkyl or aryl(C₁₋₄)alkoxy; and
optionally N substituted by trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl(C₁₋₄)alkyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl;
n is 0 or 1 and AB is NR¹¹CO, CONR¹¹, CO-CR⁸R⁹, CR⁶R⁷-CO, O-CR⁸R⁹, CR⁶R⁷-O, NHR¹¹-CR⁸R⁹, CR⁶R⁷-NHR¹¹, NR¹¹SO₂, CR⁶R⁷-SO₂ or CR⁶R⁷ CR⁸R⁹,
provided that if n=0, B is not NR¹¹, O or SO₂,
and provided that R⁶ and R⁷, and R⁸ and R⁹ are not both optionally substituted hydroxy or amino;
and wherein:
each of R⁶, R⁷ , R⁸ and R⁹ is independently selected from: H; (C₁₋₆)alkoxy; (C₁₋₆)alkylthio; halo; trifluoromethyl; azido; (C ₁₋₆)alkyl; (C₂₋₆)alkenyl; (C₁₋₆)alkoxycarbonyl; (C ₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or (C ₁₋₆)aminosulphonyl wherein the amino group is optionally substituted by (C₁₋₆)alkyl or (C₂₋₆)alkenyl;
or R⁶ and R⁸ together represent a bond and R⁷ and R⁹ are as above defined;
in optionally substituted amino the amino group is optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl;
in optionally substituted aminocarbonyl the amino group is optionally substituted by (C₁₋₆)alkyl, hydroxy(C₁₋₆)alkyl, aminocarbonyl(C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl;
and each R¹¹ is independently H; trifluoromethyl; (C₁₋₆)alkyl; (C₂₋₆)alkenyl; (C₁₋₆)alkoxycarbonyl; (C ₁₋₆)alkylcarbonyl; or aminocarbonyl wherein the amino group is optionally substituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl;
or where one of R⁶, R⁷, R⁸ or R⁹ contains a carboxy group they may together with R³ form a cyclic ester linkage.

2. A compound according to claim 1 wherein Z⁵ is CH, Z³ is CH or CF, Z¹ is CH or C-OCH₃ and Z² and Z⁴ are each CH, or Z¹ is N, Z³ is CH or CF and Z², Z⁴ and Z⁵ are each CH

3. A compound according to any preceding claim wherein R¹ is methoxy or fluoro and R^{1a} is H or when Z³ is CR^{1a} it may be C-F.

4. A compound according to any preceding claim wherein R² is hydrogen.

5. A compound according to any preceding claim wherein R³ is hydroxy.

6. A compound according to any preceding claim wherein n is 0 and either A is CHOH or CH₂ and B is CH₂ or A is NH and B is CO, and AB(CH₂)ₙ and NR²R⁴ are trans.

7. A compound according to any preceding claim wherein R⁴ is -U-R⁵₂, the group -U- is -CH₂-, and R⁵₂ is an aromatic heterocyclic ring (A) having 8-11 ring atoms including 2-4 heteroatoms of which at least one is N or NR¹³ or the heterocyclic ring (A) has ring (a) aromatic selected from optionally substituted benzo and pyrido and ring (b) non-aromatic and Y² has 3-5 atoms including NR¹³, O or S bonded to X⁵ and NHCO bonded via N to X³, or O bonded to X³.

8. A compound according to any of claims 1 to 6 wherein R⁵₂ is selected from:
benzo[1,2,5]thiadiazol-5-yl
4H-benzo[1,4] thiazin-3-one-6-yl
2,3-dihydro-benzo[1,4]dioxin-6-yl
benzo[1,2,3]thiadiazol-5-yl
3-oxo-3,4-dihydro-2H-benzo[1,4]oxazin-6-yl
7-fluoro-3-oxo-3,4-dihydro-2H-benzo[1,4]oxazin-6-yl
2-oxo-2,3-dihydro-1H-pyrido[2,3-b][1,4]thiazin-7-yl
2,3-dihydro-[1,4]dioxino[2,3-c]pyridin-7-yl
3-oxo-3,4-dihydro-2H-pyrido[3,2-b][1,4]oxazin-6-yl
[1,2,3]thiadiazolo[5,4-b]pyridin-6-yl
3-oxo-3,4-dihydro-2*H*-pyrido[3,2-*b*][1,4]thiazin-6-yl
7-chloro-3-oxo-3,4-dihydro-2*H*-pyrido[3,2-*b*][1,4]thiazin-6-yl
7-fluoro-3-oxo-3,4-dihydrn-2*H*-pyrido[3,2-*b*][1,4]thiazin-6-yl
2-oxo-2,3-dihydro-1*H*-pyrido[3,4-*b*][1,4]thiazin-7-yl.

9. A compound selected from:
(1*R*,4*S*)-1-Hydroxy-4-[(3-oxo-3,4-dihydro-2*H*-pyrido[3,2-*b*][1,4]thiazin-6-ylmethyl)-amino]-cyclohex-2-enecarboxylic acid (6-methoxy-[1,5]naphthyridin-4-yl)-amide and
(1*S*,4*R*)-1-Hydroxy-4-[(3-oxo-3,4-dihydro-2*H*-pyrido[3,2-*b*][1,4]thiazin-6-ylmethyl)-amino]-cyclohex-2-enecarboxylic acid (6-methoxy-[1,5]naphthyridin-4-yl)-amide;
(1*R*,4*S*)-1-Hydroxy-4-[(3-oxo-3,4-dihydro-2*H*-pyrido[3,2-*b*][1,4]oxazin-6-ylmethyl)-amino]-cyclohex-2-enecarboxylic acid (6-methoxy-[1,5]naphthyridin-4-yl)-amide and
(1*S*,4*R*)-1-Hydroxy-4-[(3-oxo-3,4-dihydro-2*H*-pyrido[3,2-*b*][1,4]oxazin-6-ylmethyl)-amino]-cyclohex-2-enecarboxylic acid (6-methoxy-[1,5]naphthyridin-4-yl)-amide; 1-Hydroxy-*t*-4-[(2,3-dihydro[1,4]dioxino[2,3-*c*]pyridine-7-ylmethyl)-amino]-*r-*cyclohex-2-enecarboxylic acid (6-methoxy-[1,5]naphthyridin-4-yl)-amide (E2 isomer) or a pharmaceutically acceptable derivative thereof.

10. The use of a compound according to claim 1, in the manufacture of a medicament for use in the treatment of bacterial infections in mammals.

11. A pharmaceutical composition comprising a compound according to claim 1, and a pharmaceutically acceptable carrier.

12. A process for preparing a compound according to claim 1, which process comprises reacting a compound of formula (IV) with a compound of formula (V): 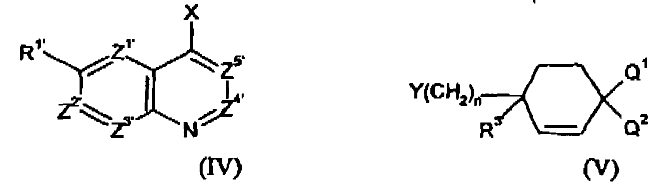 wherein n is as defined in formula (I); Z^{1'}, Z^{2'}, Z^{3'}, Z^{4'}, Z^{5'}, R^{1'} and R^{3'} are Z¹, Z², Z³, Z⁴, Z⁵, R¹ and R³ as defined in formula (I) or groups convertible thereto;
Q ¹ is NR^{2'}R^{4'} or a group convertible thereto wherein R^{2'} and R^{4'} are R² and R⁴ as defined in formula (I) or groups convertible thereto and Q² is H or R^{3'} or Q¹ and Q² together form an optionally protected oxo group;
and X and Y may be the following combinations:
(i) one of X and Y is CO₂R^{y} and the other is CH₂CO₂R^{x};
(ii) X is CHR⁶R⁷ and Y is C(=O)R⁹;
(iii) X is CR⁷=PR^{z}₃ and Y is C(=O)R⁹;
(iv) X is C(=O)R⁷ and Y is CR⁹=PR^{z}₃;
(v) one of Y and X is COW and the other is NHR^{11'};
(vi) X is NHR^{11'} and Y is C(=O)R⁸ or X is C(=O)R⁶ and Y is NHR^{11'};
(vii) X is NHR^{11'} and Y is CR⁸R⁹W;
(viii) X is W or OH and Y is CH₂OH;
(ix) X is NHR^{11'} and Y is SO₂W;
(x) one of X and Y is (CH₂)ₚ-W and the other is (CH₂)_{q}NHR^{11'}, (CH₂)_{q}OH, (CH₂)_{q}SH or (CH₂)_{q}SCOR^{x} where p+q=1;
(xi) one of X and Y is OH and the other is -CH=N₂;
(xii) X is W and Y is CONHR¹¹;
(xiii) X is W and Y is -C≡CH followed by selective reduction of the intermediate -C≡C- group;
in which W is a leaving group, e.g. halo or imidazolyl; R^{x} and R^{y} are (C₁₋₆)alkyl; R^{z} is aryl or (C₁₋₆)alkyl; A' and NR^{11'} are A and NR¹¹ as defined in formula (I), or groups convertible thereto; and oxirane is: 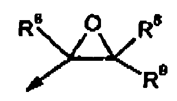 wherein R⁶, R⁸ and R⁹ are as defined in formula (I);
and thereafter optionally or as necessary converting Q¹ and Q² to NR^{2'}R^{4'}; converting A', Z^{1'}, Z^{2'}, Z^{3'}, Z^{4'}, Z^{5'}, R^{1'}, R^{2'}, R^{3'}, R^{4'} and NR^{11'} to A, Z¹, Z², Z³, NR¹¹ x Z⁴, Z⁵, R¹, R², R³, R⁴ and NR¹¹; converting A-B to other A-B, interconverting R^{v}, R^{w}, R¹, R², R³ and/or R⁴, and/or forming a pharmaceutically acceptable derivative thereof.

13. A compound of formula (VII): 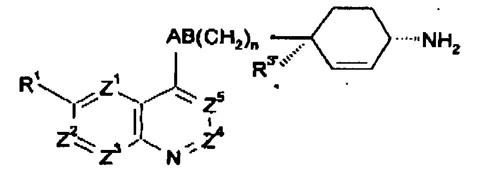 wherein the variables are as described for formula (I) in claim 1.

## Patentansprüche

1. Verbindung der Formel (I) oder pharmazeutisch verträgliches Derivat davon: 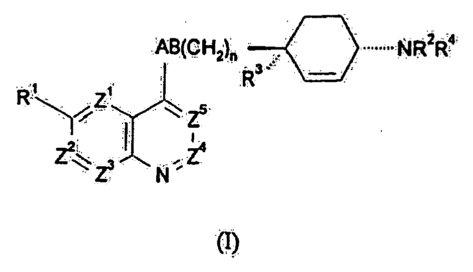 wobei:
einer der Reste Z¹, Z², Z³, Z⁴ und Z⁵ gleich N ist, ein Rest gleich CR^{1a} ist und die übrigen CH sind, oder einer der Reste Z¹, Z², Z³, Z⁴ und Z⁵ gleich CR^{1a} ist und die übrigen CH sind;
R¹ und R^{1a} unabhängig ausgewählt sind aus Wasserstoff; Hydroxy; (C₁₋₆)-Alkoxy, gegebenenfalls substituiert mit (C₁₋₆)-Alkoxy, Amino, Piperidyl, Guanidino oder Amidino, welche jeweils gegebenenfalls N-substituiert sind mit einem oder zwei (C₁₋₆)-Alkyl, Acyl oder (C₁₋₆)-Alkylsulfonylresten, CONH₂, Hydroxy, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₁₋₆)-Alkoxy-substituiertem (C₁₋₆)-Alkyl; Halogen; (C₁₋₆)-Alkyl; (C₁₋₆)-Alkylthio; Trifluormethyl; Nitro; Azido; Acyl; Acyloxy; Acylthio; (C₁₋₆)-Alkylsulfonyl; (C₁₋₆)-Alkylsulfoxid; Arylsulfonyl; Arylsulfoxid oder einem Amino-, Piperidyl-, Guanidino- oder Amidinorest, gegebenenfalls N-substituiert mit einem oder zwei (C₁₋₆)-Alkyl, Acyl oder (C₁₋₆)-Alkylsulfonylresten, oder wenn Z¹ gleich CR^{1a} ist, R¹ und R^{1a} zusammen (C₁₋₂)-Alkylendioxy sein können, oder wenn Z⁵ CR^{1a} ist, R^{1a} stattdessen Cyano, Hydroxymethyl oder Carboxy sein kann,
mit der Maßgabe, dass wenn Z¹, Z², Z³, Z⁴ und Z⁵ gleich CR^{1a} oder CH sind, dann ist R¹ nicht Wasserstoff;
R² Wasserstoff oder (C₁₋₄)-Alkyl oder (C₂₋₄)-Alkenyl ist, gegebenenfalls substituiert mit 1 bis 3 Resten, ausgewählt aus:
Amino, gegebenenfalls substituiert mit einem oder zwei (C₁₋₄)-Alkylresten; Carboxy; (C₁₋₄)-Alkoxycarbonyl; (C₁₋₄)-Alkylcarbonyl; (C₂₋₄)-Alkenyloxycarbonyl; (C₂₋₄)-Alkenylcarbonyl; Aminocarbonyl, wobei der Aminorest gegebenenfalls substituiert ist mit Hydroxy, (C₁₋₄)-Alkyl, Hydroxy(C₁₋₄)-alkyl, Aminocarbonyl(C₁₋₄)-alkyl, (C₂₋₄)-Alkenyl, (C₁₋₄)-Alkylsulfonyl, Trifluormethylsulfonyl, (C₂₋₄)-Alkenylsulfonyl, (C₁₋₄)-Alkoxycarbonyl, (C₁₋₄)-Alkylcarbonyl, (C₂₋₄)-Alkenyloxycarbonyl oder (C₂₋₄)-Alkenylcarbonyl; Cyano; Tetrazolyl; 2-Oxo-oxazolidinyl, gegebenenfalls substituiert mit R¹⁰; 3-Hydroxy-3-cyclobuten-1,2-dion-4-yl; 2,4-Thiazolidindion-5-yl; Tetrazol-5-ylaminocarbonyl; 1,2,4-Triazol-5-yl, gegebenenfalls substituiert mit R¹⁰; 5-Oxo-1,2,4-oxadiazol-3-yl; Halogen; (C₁₋₄)-Alkylthio; Trifluormethyl; Hydroxy, gegebenenfalls substituiert mit (C₁₋₄)-Alkyl, (C₂₋₄)-Alkenyl, (C₁₋₄)-Alkoxycarbonyl, (C₁₋₄)-Alkylcarbonyl, (C₂₋₄)-Alkenyloxycarbonyl, (C₂₋₄)-Alkenylcarbonyl; Oxo; (C₁₋₄)-Alkylsulfonyl; (C₂₋₄)-Alkenylsulfonyl; oder (C₁₋₄)-Aminosulfonyl, wobei der Aminorest gegebenenfalls mit (C₁₋₄)-Alkyl oder (C₂₋₄)-Alkenyl substituiert ist;
R³ Hydroxy ist, gegebenenfalls substituiert mit (C₁₋₆)-Alkyl, (C₂₋₆)-Alkenyl, (C₁₋₆)-Alkoxycarbonyl, (C₁₋₆)-Alkylcarbonyl, (C₂₋₆)-Alkenyloxycarbonyl, (C₂₋₆)-Alkenylcarbonyl oder Aminocarbonyl, wobei der Aminorest gegebenenfalls substituiert ist mit (C₁₋₆)-Alkyl, (C₂₋₆)-Alkenyl, (C₁₋₆)-Alkylcarbonyl oder (C₂₋₆)-Alkenylcarbonyl; R¹⁰ ausgewählt ist aus (C₁₋₄)-Alkyl und (C₂₋₄)-Alkenyl, welche jeweils gegebenenfalls mit einem Rest R¹² wie vorstehend definiert substituiert sein können; Carboxy; Aminocarbonyl, wobei der Aminorest gegebenenfalls substituiert ist mit Hydroxy, (C₁₋₆)-Alkyl, (C₂₋₆)-Alkenyl, (C₁₋₆)-Alkylsulfonyl, Trifluormethylsulfonyl, (C₂₋₆)-Alkenylsulfonyl, (C₁₋₆)-Alkoxycarbonyl, (C₁₋₆)-Alkylcarbonyl, (C₂₋₆)-Alkenyloxycarbonyl oder (C₂₋₆)-Alkenylcarbonyl und gegebenenfalls weiter substituiert ist mit (C₁₋₆)-Alkyl oder (C₂₋₆)-Alkenyl; (C₁₋₆)-Alkylsulfonyl; Trifluormethylsulfonyl; (C₂₋₆)-Alkenylsulfonyl; (C₁₋₆)-Alkoxycarbonyl; (C₁₋₆)-Alkylcarbonyl; (C₂₋₆)-Alkenyloxycarbonyl; und (C₂₋₆)-Alkenylcarbonyl;
R⁴ ein Rest -CH₂-R⁵₁ ist, in welchem R⁵₁ ausgewählt ist aus:
(C₄₋₈)-Alkyl; Hydroxy(C₄₋₈)-alkyl; (C₁₋₄)-Alkoxy(C₄₋₈)-alkyl; (C₁₋₄)-Alkanoyloxy(C₄₋₈)-alkyl; (C₃₋₈)-Cycloalkyl(C₄₋₈)-alkyl; Hydroxy-, (C₁₋₆)-Alkoxy- oder (C₁₋₆)-Alkanoyloxy-(C₃₋₈)-cycloalkyl(C₄₋₈)-alkyl; Cyano(C₄₋₈)-alkyl; (C₄₋₈)-Alkenyl; (C₄₋₈)-Alkinyl; Tetrahydrofuryl; Mono- oder Di-(C₁₋₆)-alkylamino(C₄₋₈)-alkyl; Acylamino(C₄₋₈)-alkyl; (C₁₋₆)-Alkyl- oder Acyl-aminocarbonyl(C₄₋₈)-alkyl; Mono- oder Di(C₁₋₆)-alkylamino(hydroxy)(C₄₋₈)-alkyl; oder
R⁴ ein Rest -U-R⁵₂ ist, wobei R⁵₂ ein gegebenenfalls substituiertes, bicyclisches, carbocyclisches oder heterocyclisches Ringsystem (A) ist: 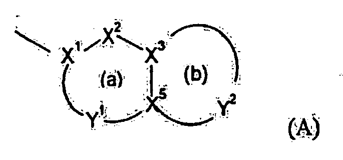 das bis zu vier Heteroatome in jedem Ringsystem enthält, wobei
mindestens einer der Ringe (a) und (b) aromatisch ist;
X¹ gleich C oder N ist, wenn es Teil eines aromatischen Rings ist, oder CR¹⁴ ist, wenn es Teil eines nichtaromatischen Rings ist;
X² gleich N, NR¹³, O, S(O)ₓ, CO oder CR¹⁴ ist, wenn es Teil eines aromatischen oder nichtaromatischen Rings ist, oder zusätzlich CR¹⁴R¹⁵ sein kann, wenn es Teil eines nichtaromatischen Rings ist;
X³ und X⁵ unabhängig N oder C sind;
Y¹ eine Linkergruppe mit 0 bis 4 Atomen ist, wobei jedes Atom unabhängig ausgewählt ist aus N, NR¹³, O, S(O)ₓ, CO und CR¹⁴, wenn es Teil eines aromatischen oder nichtaromatischen Rings ist, oder zusätzlich CR¹⁴R¹⁵ sein kann, wenn es Teil eines nichtaromatischen Rings ist,
Y² eine Linkergruppe mit 2 bis 6 Atomen ist, wobei jedes Atom von Y² unabhängig ausgewählt ist aus N, NR¹³, O, S(O)ₓ, CO und CR¹⁴, wenn es Teil eines aromatischen oder nichtaromatischen Rings ist, oder zusätzlich CR¹⁴R¹⁵ sein kann, wenn es Teil eines nichtaromatischen Rings ist;
R¹⁴ und R¹⁵ jeweils unabhängig ausgewählt sind aus: H; (C₁₋₄)-Alkylthio; Halogen; Carboxy(C₁₋₄)-alkyl; Halogen(C₁₋₄)-alkoxy; Halogen(C₁₋₄)-alkyl; (C₁₋₄)-Alkyl; (C₂₋₄)-Alkenyl; (C₁₋₄)-Alkoxycarbonyl; Formyl; (C₁₋₄)-Alkylcarbonyl; (C₂₋₄)-Alkenyloxycarbonyl; (C₂₋₄)-Alkenylcarbonyl; (C₁₋₄)-Alkylcarbonyloxy; (C₁₋₄)-Alkoxycarbonyl(C₁₋₄)-alkyl;
Hydroxy; Hydroxy(C₁₋₄)-alkyl; Mercapto(C₁₋₄)-alkyl; (C₁₋₄)-Alkoxy; Nitro; Cyano; Carboxy; Amino oder Aminocarbonyl, gegebenenfalls substituiert wie für entsprechende Substituenten in R³; (C₁₋₄)-Alkylsulfonyl; (C₂₋₄)-Alkenylsulfonyl; oder Aminosulfonyl, wobei der Aminorest gegebenenfalls substituiert ist mit (C₁₋₄)-Alkyl oder (C₂₋₄)-Alkenyl; Aryl; Aryl(C₁₋₄)-alkyl; Aryl(C₁₋₄)-alkoxy;
R¹³ jeweils unabhängig H; Trifluormethyl; (C₁₋₄)-Alkyl, gegebenenfalls substituiert mit Hydroxy, Carboxy, (C₁₋₆)-Alkoxycarbonyl, (C₁₋₆)-Alkoxy, (C₁₋₆)-Alkylthio, Halogen oder Trifluormethyl; (C₂₋₄)-Alkenyl; Aryl; Aryl(C₁₋₄)-alkyl; Arylcarbonyl; Heteroarylcarbonyl; (C₁₋₄)-Alkoxycarbonyl; (C₁₋₄)-Alkylcarbonyl; Formyl; (C₁₋₆)-Alkylsulfonyl; oder Aminocarbonyl ist, wobei der Aminorest gegebenenfalls substituiert ist mit (C₁₋₄)-Alkoxycarbonyl, (C₁₋₄)-Alkylcarbonyl, (C₂₋₄)-Alkenyloxycarbonyl, (C₂₋₄)-Alkenylcarbonyl, (C₁₋₄)-Alkyl oder (C₂₋₄)-Alkenyl und gegebenenfalls weiterhin substituiert ist mit (C₁₋₄)-Alkyl oder (C₂₋₄)-Alkenyl;
x jeweils unabhängig 0, 1 oder 2 ist;
U gleich CO, SO₂ oder CH₂ ist; oder
R⁴ ein Rest -X^{1a}-X^{2a}-X^{3a}-X^{4a} ist, wobei:
X^{1a} gleich CH₂, CO oder SO₂ ist;
X^{2a} gleich CR^{14a}R^{15a} ist;
X^{3a} gleich NR^{13a}, O, S, SO₂ oder CR^{14a}R^{15a} ist, wobei:
R^{14a} und R^{15a} jeweils unabhängig ausgewählt sind aus den vorstehend für R¹⁴ und R¹⁵ angegebenen Resten, mit der Maßgabe, dass R^{14a} und R^{15a} am gleichen Kohlenstoffatom nicht beide aus gegebenenfalls substituiertem Hydroxy und gegebenenfalls substituiertem Amino ausgewählt sind; oder
R^{14a} und R^{15a} zusammen Oxo bedeuten;
R^{13a} Wasserstoff; Trifluormethyl; (C₁₋₆)-Alkyl; (C₂₋₆)-Alkenyl; (C₁₋₆)-Alkoxycarbonyl; (C₁₋₆)-Alkylcarbonyl; oder Aminocarbonyl ist, wobei der Aminorest gegebenenfalls substituiert ist mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl oder (C₂₋₆)-Alkenyl und gegebenenfalls weiter substituiert ist mit (C₁₋₆)-Alkyl oder (C₂₋₆)-Alkenyl; oder
zwei Reste R^{14a} oder ein Rest R^{13a} und ein Rest R^{14a} an benachbarten Atomen zusammen eine Bindung bedeuten und die übrigen Reste R^{13a}, R^{14a} und R^{15a} wie vorstehend definiert sind; oder
zwei Reste R^{14a} und zwei Reste R^{15a} an benachbarten Atomen zusammen Bindungen bedeuten, so dass X^{2a} und X^{3a} dreifach gebunden ist;
X^{4a} Phenyl oder ein über C oder N gebundener, monocyclischer, aromatischer, 5- oder 6-gliedriger Heterocyclus ist, der bis zu vier Heteroatome, ausgewählt aus O, S und N, enthält und:
gegebenenfalls am C substituiert ist mit bis zu drei Reste, ausgewählt aus (C₁₋₄)-Alkylthio; Halogen; Carboxy(C₁₋₄)-alkyl; Halogen(C₁₋₄)-alkoxy; Halogen(C₁₋₄)-alkyl; (C₁₋₄)-Alkyl; (C₂₋₄)-Alkenyl; (C₁₋₄)-Alkoxycarbonyl; Formyl; (C₁₋₄)-Alkylcarbonyl; (C₂₋₄)-Alkenyloxycarbonyl; (C₂₋₄)-Alkenylcarbonyl; (C₁₋₄)-Alkylcarbonyloxy; (C₁₋₄)-Alkoxycarbonyl(C₁₋₄)-alkyl; Hydroxy; Hydroxy(C₁₋₄)-alkyl; Mercapto(C₁₋₄)-alkyl; (C₁₋₄)-Alkoxy; Nitro; Cyano; Carboxy; Amino oder Arminocarbonyl, gegebenenfalls substituiert wie für entsprechende Substituenten in R³; (C₁₋₄)-Alkylsulfonyl; (C₂₋₄)-Alkenylsulfonyl; oder Aminosulfonyl, wobei der Aminorest gegebenenfalls substituiert ist mit (C₁₋₄)-Alkyl oder (C₂₋₄)-Alkenyl; Aryl, Aryl(C₁₋₄)-alkyl oder Aryl(C₁₋₄)-alkoxy; und
gegebenenfalls am N substituiert ist mit Trifluormethyl; (C₁₋₄)-Alkyl, gegebenenfalls substituiert mit Hydroxy, (C₁₋₆)-Alkoxy, (C₁₋₆)-Alkylthio, Halogen oder Trifluormethyl; (C₂₋₄)-Alkenyl; Aryl; Aryl(C₁₋₄)-alkyl; (C₁₋₄)-Alkoxycarbonyl; (C₁₋₄)-Alkylcarbonyl; Formyl; (C₁₋₆)-Alkylsulfonyl; oder Aminocarbonyl, wobei der Aminorest gegebenenfalls substituiert ist mit (C₁₋₄)-Alkoxycarbonyl, (C₁₋₄)-Alkylcarbonyl, (C₂₋₄)-Alkenyloxycarbonyl, (C₂₋₄)-Alkenylcarbonyl, (C₁₋₄)-Alkyl oder (C₂₋₄)-Alkenyl und gegebenenfalls weiter substituiert ist mit (C₁₋₄)-Alkyl oder (C₂₋₄)-Alkenyl;
n 0 oder 1 ist und AB gleich NR¹¹CO, CONR¹¹, CO-CR⁸R⁹, CR⁶R⁷-CO, O-CR⁸R⁹, CR⁶R⁷-O, NHR¹¹-CR⁸R⁹, CR⁶R⁷-NHR¹¹, NR¹¹SO₂, CR⁶R⁷-SO₂ oder CR⁶R⁷-CR⁸R⁹ ist,
mit der Maßgabe, dass, wenn n=0, B nicht NR¹¹, O oder SO₂ ist,
und mit der Maßgabe, dass R⁶ und R⁷, und R⁸ und R⁹ nicht beide gegebenenfalls substituiertes Hydroxy oder Amino sind;
und wobei:
R⁶, R⁷, R⁸ und R⁹ jeweils unabhängig ausgewählt sind aus: H; (C₁₋₆)-Alkoxy; (C₁₋₆)-Alkylthio; Halogen; Trifluormethyl; Azido; (C₁₋₆)-Alkyl; (C₂₋₆)-Alkenyl; (C₁₋₆)-Alkoxycarbonyl; (C₁₋₆)-Alkylcarbonyl; (C₂₋₆)-Alkenyloxycarbonyl; (C₂₋₆)-Alkenylcarbonyl; Hydroxy, Amino oder Aminocarbonyl, gegebenenfalls substituiert wie für entsprechende Substituenten in R³; (C₁₋₆)-Alkylsulfonyl; (C₂₋₆)-Alkenylsulfonyl; oder (C₁₋₆)-Aminosulfonyl, wobei der Aminorest gegebenenfalls substituiert ist mit (C₁₋₆)-Alkyl oder (C₂₋₆)-Alkenyl;
oder R⁶ und R⁸ zusammen eine Bindung bedeuten und R⁷ und R⁹ wie vorstehend definiert sind;
in gegebenenfalls substituiertem Amino der Aminorest gegebenenfalls mono- oder disubstituiert ist mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl, (C₂₋₆)-Alkenyl, (C₁₋₆)-Alkylsulfonyl, (C₂₋₆)-Alkenylsulfonyl oder Aminocarbonyl, wobei der Aminorest gegebenenfalls substituiert ist mit (C₁₋₆)-Alkyl oder (C₂₋₆)-Alkenyl;
in gegebenenfalls substituiertem Aminocarbonyl der Aminorest gegebenenfalls substituiert ist mit (C₁₋₆)-Alkyl, Hydroxy(C₁₋₆)-alkyl, Aminocarbonyl(C₁₋₆)-alkyl, (C₂₋₆)-Alkenyl, (C₁₋₆)-Alkoxycarbonyl, (C₁₋₆)-Alkylcarbonyl, (C₂₋₆)-Alkenyloxycarbonyl oder (C₂₋₆)-Alkenylcarbonyl und gegebenenfalls weiter substituiert ist mit (C₁₋₆)-Alkyl, Hydroxy(C₁₋₆)-alkyl, Aminocarbonyl(C₁₋₆)-alkyl oder (C₂₋₆)-Alkenyl;
und R¹¹ jeweils unabhängig H; Trifluormethyl; (C₁₋₆)-Alkyl; (C₂₋₆)-Alkenyl; (C₁₋₆)-Alkoxycarbonyl; (C₁₋₆)-Alkylcarbonyl; oder Aminocarbonyl ist, wobei der Aminorest gegebenenfalls substituiert ist mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl oder (C₂₋₆)-Alkenyl und gegebenenfalls weiter substituiert ist mit (C₁₋₆)-Alkyl oder (C₂₋₆)-Alkenyl;
oder, wenn ein Rest R⁶, R⁷, R⁸ oder R⁹ eine Carboxygruppe enthält, sie zusammen mit R³ eine cyclische Esterverbindung bilden können.

2. Verbindung gemäß Anspruch 1, wobei Z⁵ gleich CH ist, Z³ gleich CH oder CF ist, Z¹ gleich CH oder C-OCH₃ ist und Z² und Z⁴ jeweils CH sind, oder Z¹ gleich N ist, Z³ gleich CH oder CF ist und Z², Z⁴ und Z⁵ jeweils CH sind.

3. Verbindung gemäß einem der vorstehenden Ansprüche, wobei R¹ Methoxy oder Fluor ist und R^{1a} gleich H ist oder wenn Z³ gleich CR^{1a} ist, es C-F sein kann.

4. Verbindung gemäß einem der vorstehenden Ansprüche, wobei R² Wasserstoff ist.

5. Verbindung gemäß einem der vorstehenden Ansprüche, wobei R³ Hydroxy ist.

6. Verbindung gemäß einem der vorstehenden Ansprüche, wobei n 0 ist und entweder A gleich CHOH oder CH₂ ist und B gleich CH₂ ist oder A gleich NH ist und B gleich CO ist, und AB(CH₂)ₙ und NR²R⁴ trans stehen.

7. Verbindung gemäß einem der vorstehenden Ansprüche, wobei R⁴ gleich U-R⁵₂ ist, der Rest -U- gleich -CH₂- ist, und R⁵₂ ein aromatischer heterocyclischer Ring (A) mit 8-11 Ringatomen ist, der 2-4 Heteroatome einschließt, von welchen mindestens eines N oder NR¹³ ist, oder der heterocyclische Ring (A) einen aromatischen Ring (a), ausgewählt aus gegebenenfalls substituiertem Benzo und Pyrido, und einen nichtaromatischen Ring (b) aufweist, und Y² 3-5 Atome aufweist, einschließend NR¹³, O oder S, gebunden an X⁵, und NHCO, gebunden über N an X³, oder O, gebunden an X³.

8. Verbindung gemäß einem der Ansprüche 1 bis 6, wobei R⁵₂ ausgewählt ist aus:
Benzo[1,2,5]thiadiazol-5-yl
4H-Benzo[1,4]thiazin-3-on-6-yl
2,3-Dihydro-benzo[1,4]dioxin-6-yl
Benzo[1,2,3]thiadiazol-5-yl
3-Oxo-3,4-dihydro-2H-benzo[1,4]oxazin-6-yl
7-Fluor-3-oxo-3,4-dihydro-2H-benzo[1,4]oxazin-6-yl
2-Oxo-2,3-dihydro-1H-pyrido[2,3-b][ 1,4]thiazin-7-yl
2,3-Dihydro-[1,4]dioxino[2,3-c]pyridin-7-yl
3-Oxo-3,4-dihydro-2H-pyrido[3,2-b][1,4]oxazin-6-yl
[1,2,3]Thiadiazolo[5,4-b]pyridin-6-yl
3-Oxo-3,4-dihydro-2H-pyrido[3,2-b][1,4]thiazin-6-yl
7-Chlor-3-oxo-3,4-dihydro-2H-pyrido[3,2-b][1,4]thiazin-6-yl
7-Fluor-3-oxo-3,4-dihydro-2H-pyrido[3,2-b][1,4]thiazin-6-yl
2-Oxo-2,3-dihydro-1H-pyrido[3,4-b][1,4]thiazin-7-yl.

9. Verbindung, ausgewählt aus:
(1R,4S)-1-Hydroxy-4-[(3-oxo-3,4-dihydro-2H-pyrido[3,2-b][1,4]thiazin-6-ylmethyl)-amino]-cyclohex-2-encarbonsäure (6-methoxy-[1,5]naphthyridin-4-yl)-amid und
(1S,4R)-1-Hydroxy-4-[(3-oxo-3,4-dihydro-2H-pyrido[3,2-b][1,4]thiazin-6-ylmethyl)-amino]-cyclohex-2-encarbonsäure (6-methoxy-[1,5]naphthyridin-4-yl)amid;
(1R,4S)-1-Hydroxy-4-[(3-oxo-3,4-dihydro-2H-pyrido[3,2-b][1,4]oxazin-6-ylmethyl)-amino]-cylohex-2-encarbonsäure (6-methoxy-[1,5]naphthyridin-4-yl)-amid und
(1S,4R)-1-Hydroxy-4-[(3-oxo-3,4-dihydro-2H-pyrido[3,2-b][1,4]oxazin-6-ylmethyl)-amino]-cyclohex-2-encarbonsäure (6-methoxy-[1,5]naphthyridin-4-yl)-amid;
1-Hydroxy-t-4-[(2,3-dihydro[1,4]dioxino[2,3-c]pyridin-7-ylmethyl)-amino]-r-cyclohex-2-encarbonsäure (6-methoxy-[1,5]naphthyridin-4-yl)-amid (E2-Isomer) oder ein pharmazeutisch verträgliches Derivat davon.

10. Verwendung einer Verbindung gemäß Anspruch 1 zur Herstellung eines Medikaments zur Verwendung bei der Behandlung von bakteriellen Infektionen in Säugern.

11. Arzneimittel, umfassend eine Verbindung gemäß Anspruch 1 und einen pharmazeutisch verträglichen Träger.

12. Verfahren zur Herstellung einer Verbindung gemäß Anspruch 1, wobei das Verfahren umfasst:
Umsetzen einer Verbindung der Formel (IV) mit einer Verbindung der Formel (V): 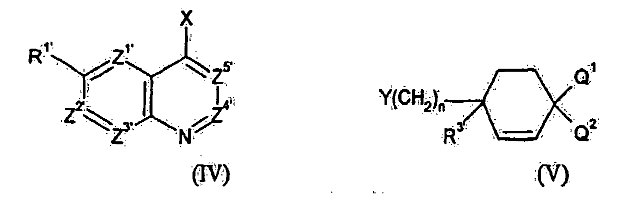
wobei n wie in Formel (I) definiert ist; Z^{1'}, Z^{2'}, Z^{3'}, Z^{4'}, Z^{5'}, R^{1'} und R^{3'} gleich Z¹, Z², Z³, Z⁴, Z⁵, R¹ und R³ wie in Formel (I) definiert oder dazu umwandelbare Rest sind;
Q¹ gleich NR^{2'}R^{4'} oder ein dazu umwandelbarer Rest ist, wobei R^{2'} und R^{4'} gleich R² und
R⁴ wie in Formel (I) definiert oder dazu umwandelbare Reste sind, und Q² gleich H oder R^{3'} ist oder Q¹ und Q² zusammen einen gegebenenfalls geschützten Oxorest bilden;
und X und Y die folgenden Kombinationen sein können:
(i) einer der Reste X und Y ist CO₂R^{y} und der andere Rest ist CH₂CO₂R^{x};
(ii) X ist CHR⁶R⁷ und Y ist C(=O)R⁹,
(iii) X ist CR⁷=PR^{z}₃ und Y ist C(=O)R⁹;
(iv) X ist C(=O)R⁷ und Y ist CR⁹=PR^{z}₃;
(v) einer der Reste Y und X ist COW und der andere Rest ist NHR^{11'};
(vi) X ist NHR^{11'} und Y ist C(=O)R⁸ oder X ist C(=O)R⁶ und Y ist NHR^{11'};
(vii) X ist NHR^{11'} und Y ist CR⁸R⁹W;
(viii) X ist W oder OH und Y ist CH₂OH;
(ix) X ist NHR^{11'} und Y ist SO₂W;
(x) einer der Reste X und Y ist (CH₂)ₚ-W und der andere Rest ist (CH₂)_{q}NHR^{11'}, (CH₂)_{q}OH, (CH₂)_{q}SH oder (CH₂)_{q}SCOR^{x}, wobei p+q=1;
(xi) einer der Reste X und Y ist OH und der andere Rest ist -CH=N₂;
(xii) X ist W und Y ist CONHR¹¹;
(xiii) X ist W und Y ist -C≡CH, gefolgt von selektiver Reduktion des Zwischenprodukts -C≡C-;
wobei W eine Abgangsgruppe ist, z.B. Halogen oder Imidazolyl; R^{x} und R^{y} (C₁₋₆)-Alkyl sind; R^{z} Aryl oder (C₁₋₆)-Alkyl ist; A' und NR^{11'} gleich A und NR¹¹ wie in Formel (I) definiert oder dazu umwandelbare Reste sind; und Oxiran: 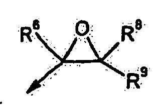 ist, wobei R⁶, R⁸ und R⁹ wie in Formel (I) definiert sind;
und danach gegebenenfalls oder falls notwendig Umwandeln von Q¹ und Q² in NR^{2'}R^{4'}; Umwandeln von A', Z^{1'}, Z^{2'}, Z^{3'}, Z^{4'}, Z^{5'}, R^{1'}, R^{2'}, R^{3'}, R^{4'} und NR^{11'} in A, Z¹, Z², Z³, Z⁴, Z⁵, R¹, R², R³, R⁴ und NR¹¹; Umwandeln von A-B in andere A-B, Ineinanderumwandeln von R^{v}, R^{w}, R¹, R², R³ und/oder R⁴, und/oder Bilden eines pharmazeutisch verträglichen Derivats davon.

13. Verbindung der Formel (VII): 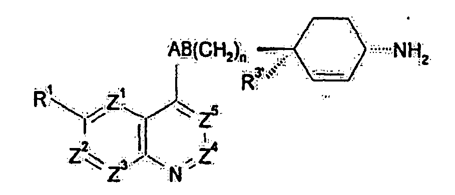 wobei die Variablen wie für Formel (I) in Anspruch 1 beschrieben sind.

## Revendications

1. Composé de formule (I) ou un de ses dérivés pharmaceutiquement acceptables : 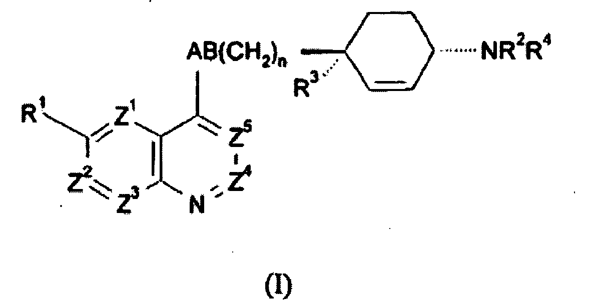 formule dans laquelle :
un de Z¹, Z², Z³, Z⁴ et Z⁵ représente N, un représente un groupe CR^{1a} et les groupes restants représentent des groupes CH, ou bien un de Z¹, Z², Z³, Z⁴ et Z⁵ représente un groupe CR^{1a} et le reste représente des groupes CH ;
R¹ et R^{1a} sont choisis indépendamment entre un atome d'hydrogène ; des groupes hydroxy ; alkoxy en C₁ à C₆ facultativement substitué avec un substituant alkoxy en C₁ à C₆, amino, pipéridyle, guanidino ou amidino dont n'importe lequel est facultativement N-substitué avec un ou deux groupes alkyle en C₁ à C₆, acyle ou alkylsulfonyle en C₁ à C₆, CONH₂, hydroxy, alkylthio en C₁ à C₆, hétérocyclylthio, hétérocyclyloxy, arylthio, aryloxy, acylthio, acyloxy ou alkylsulfonyloxy en C₁ à C₆ ; alkyle en C₁ à C₆ à substituant alkoxy en C₁ à C₆ ; halogéno ; alkyle en C₁ à C₆ ; alkylthio en C₁ à C₆ ; trifluorométhyle ; nitro ; azido ; acyle ; acyloxy ; acylthio ; alkylsulfonyle en C₁ à C₆ ; alkylsulfoxyde en C₁ à C₆ ; arylsulfonyle ; arylsulfoxyde ou un groupe amino, pipéridyle, guanidino ou amidino facultativement N-substitué avec un ou deux groupes alkyle en C₁ à C₆, acyle ou alkylsulfonyle en C₁ à C₆ ou bien, lorsque Z¹ représente un groupe CR^{1a}, R¹ et R^{1a} peuvent représenter conjointement un groupe alkylènedioxy en C₁ ou C₂ ou, lorsque Z⁵ représente un groupe CR^{1a}, R^{1a} peut représenter en variante un groupe cyano, hydroxyméthyle ou carboxy,
sous réserve que, lorsque Z¹, Z², Z³, Z⁴ et Z⁵ représentent des groupes CR^{1a} ou CH, alors R¹ ne représente pas un atome d'hydrogène ;
R² représente un atome d'hydrogène, ou un groupe alkyle en C₁ à C₄ ou alcényle en C₂ à C₄ facultativement substitué avec 1 à 3 groupes choisis entre des groupes :
amino facultativement substitué avec un ou deux groupes alkyle en C₁ à C₄ ; carboxy ; (alkoxy en C₁ à C₄)-carbonyle ; (alkyle en C₁ à C₄)carbonyle ; (alcényle en C₂ à C₄)oxycarbonyle ; (alcényle en C₂ à C₄)carbonyle ; aminocarbonyle dans lequel le groupe amino est facultativement substitué avec un substituant hydroxy, alkyle en C₁ à C₄, hydroxyalkyle en C₁ à C₄, aminocarbonyl(alkyle en C₁ à C₄) , alcényle en C₂ à C₄, alkylsulfonyle en C₁ à C₄, trifluorométhylsulfonyle, alcénylsulfonyle en C₂ à C₄, (alkoxy en C₁ à C₄)carbonyle, (alkyle en C₁ à C₄) carbonyle, (alcényle en C₂ à C₄) - oxycarbonyle ou (alcényle en C₂ à C₄)carbonyle ; cyano ; tétrazolyle ; 2-oxo-oxazolidinyle facultativement substitué avec R¹⁰ ; 3-hydroxy-3-cyclobutène-1,2-dione-4-yle ; 2,4-thiazolidinedione-5-yle ; tétrazole-5-ylaminocarbonyle ; 1,2,4-triazole-5-yle facultativement substitué avec R¹⁰ ; 5-oxo-1,2,4-oxadiazole-3-yle ; halogéno ; alkylthio en C₁ à C₄ ; trifluorométhyle ; hydroxy facultativement substitué avec un substituant alkyle en C₁ à C₄, alcényle en C₂ à C₄, (alkoxy en C₁ à C₄)carbonyle, (alkyle en C₁ à C₄)carbonyle, (alcényle en C₂ à C₄)oxycarbonyle ; (alcényle en C₂ à C₄)-carbonyle ; oxo ; alkylsulfonyle en C₁ à C₄ ; alcénylsulfonyle en C₂ à C₄ ; ou aminosulfonyle en C₁ à C₄ dans lequel le groupe amino est facultativement substitué avec un substituant alkyle en C₁ à C₄ ou alcényle en C₂ à C₄ ;
R³ représente un groupe hydroxy facultativement substitué avec un substituant alkyle en C₁ à C₆, alcényle en C₂ à C₆, (alkoxy en C₁ à C₆)carbonyle, (alkyle en C₁ à C₆)-carbonyle, (alcényle en C₂ à C₆)oxycarbonyle, (alcényle en C₂ à C₆)carbonyle ou aminocarbonyle dans lequel le groupe amino est facultativement substitué avec un substituant alkyle en C₁ à C₆, alcényle en C₂ à C₆, (alkyle en C₁ à C₆)-carbonyle ou (alcényle en C₂ à C₆)carbonyle ;
R¹⁰ est choisi entre des groupes alkyle en C₁ à C₄ et alcényle en C₂ à C₄, dont chacun peut être facultativement substitué avec un groupe R¹² répondant à la définition précitée ; carboxy ; aminocarbonyle dans lequel le groupe amino est facultativement substitué avec un substituant hydroxy, alkyle en C₁ à C₆, alcényle en C₂ à C₆, alkylsulfonyle en C₁ à C₆, trifluorométhylsulfonyle, alcénylsulfonyle en C₂ à C₆, (alkoxy en C₁ à C₆)carbonyle, (alkyle en C₁ à C₆)carbonyle, (alcényle en C₂ à C₆)-oxycarbonyle ou (alcényle en C₂ à C₆)carbonyle et est facultativement substitué en outre avec un substituant alkyle en C₁ à C₆ ou alcényle en C₂ à C₆ ; alkylsulfonyle en C₁ à C₆ ; trifluorométhylsulfonyle ; alcénylsulfonyle en C₂ à C₆ ; (alkoxy en C₁ à C₆)carbonyle ; (alkyle en C₁ à C₆)-carbonyle ; (alcényle en C₂ à C₆)oxycarbonyle et (alcényle en C₂ à C₆)-carbonyle ;
R⁴ représente un groupe -CH₂R⁵₁ dans lequel R⁵₁ est choisis entre des groupes :
alkyle en C₄ à C₈ ; hydroxyalkyle en C₄ en C₈ ; (alkoxy en C₁ à C₄) (alkyle en C₄ en C₈) ; (alcanoyle en C₁ à C₄)-oxy(alkyle en C₄ en C₈) ; (cycloalkyle en C₃ à C₈) (alkyle en C₄ en C₈) ; hydroxy-, (alkoxy en C₁ à C₆)- ou (alcanoyle en C₁ à C₆)oxy-(cycloalkyle en C₃ à C₈) (alkyle en C₄ en C₈) ; cyano (alkyle en C₄ en C₈) ; alcényle en C₄ en C₈) ; alcynyle en C₄ en C₈ ; tétrahydrofuryle ; mono- ou di-(alkyle en C₁ à C₆)amino (alkyle en C₄ en C₈) ; acylamino(alkyle en C₄ en C₈) ; (alkyle en C₁ à C₆)- ou acyl-aminocarbonyl (alkyle en C₄ en C₈) ; mono- ou di- (alkyle en C₁ à C₆) amino (hydroxy) (alkyle en C₄ en C₈) ; ou
R⁴ représente un groupe -U-R⁵₂ dans lequel R⁵₂ représente un système de noyau carbocyclique ou hétérocyclique bicyclique, facultativement substitué, (A) : 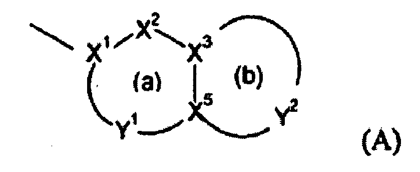 contenant jusqu'à quatre hétéroatomes dans chaque noyau, dans lequel
au moins un des noyaux (a) et (b) est aromatique ;
X¹ représente C ou N lorsqu'il fait partie d'un noyau aromatique, ou un groupe CR¹⁴ lorsqu'il fait partie d'un noyau non aromatique ;
X² représente N, un groupe NR¹³, O, S(O)ₓ, CO ou CR¹⁴ lorsqu'il fait partie d'un noyau aromatique ou non aromatique ou peut représenter en outre un groupe CR¹⁴R¹⁵ lorsqu'il fait d'un noyau non arbmatique ;
X³ et X⁵ représentent indépendamment N ou C ;
Y¹ représente un groupe de liaison de 0 à 4 atomes dont chacun est choisi indépendamment entre N, NR¹³, O, S(O)ₓ, CO et CR¹⁴ lorsqu'il fait partie d'un noyau aromatique ou non aromatique ou peut en outre représenter un groupe CR¹⁴R¹⁵ lorsqu'il fait partie d'un noyau non aromatique ;
Y² représente un groupe de liaison de 2 à 6 atomes, chaque atome de Y² étant choisi indépendamment entre N, NR¹³, O, S(O)ₓ, CO et CR¹⁴ lorsqu'il fait partie d'un noyau aromatique ou non aromatique ou peut représenter en outre un groupe CR¹⁴R¹⁵ lorsqu'il fait partie d'un noyau non aromatique, chacun de R¹⁴ et R¹⁵ est choisi indépendamment entre : H ; des groupes alkylthio en C₁ à C₄ ; halogéno ; carboxy (alkyle en C₁ à C₄) ; halogénalkoxy en C₁ à C₄ ; halogénalkyle en C₁ à C₄ ; alkyle en C₁ à C₄ ; alcényle en C₂ à C₄ ; (alkoxy en C₁ à C₄)carbonyle ; formyle ; (alkyle en C₁ à C₄)carbonyle ; (alcényle en C₂ à C₄)oxycarbonyle ; (alcényle en C₂ à C₄)carbonyle ; (alkyle en C₁ à C₄)carbonyloxy ; (alkoxy en C₁ à C₄)carbonyl(alkyle en C₁ à C₄) ; hydroxy ; hydroxyalkyle en C₁ à C₄ ; mercaptoalkyle en C₁ à C₄) ; alkoxy en C₁ à C₄ ; nitro ; cyano ; carboxy ; amino ou aminocarbonyle facultativement substitué comme pour les substituants correspondants dans R³ ; alkylsulfonyle en C₁ à C₄ ; alcénylsulfonyle en C₂ à C₄ ; ou aminosulfonyle dans lequel le groupe amino est facultativement substitué avec un substituant alkyle en C₁ à C₄ ou alcényle en C₂ à C₄ ; aryle ; aryl (alkyle en C₁ à C₄) ; ou aryl(alkoxy en C₁ à C₄) ;
chaque groupe R¹³ représente indépendamment H ; un groupe trifluorométhyle ; alkyle en C₁ à C₄ facultativement substitué avec un substituant hydroxy, carboxy, (alkoxy en C₁ à C₆)carbonyle, alkoxy en C₁ à C₆, alkylthio en C₁ à C₆, halogéno ou trifluorométhyle ; alcényle en C₂ à C₄ ; aryle ; aryl(alkyle en C₁ à C₄) ; arylcarbonyle ; hétéroarylcarbonyle ; (alkoxy en C₁ à C₄)carbonyle ; (alkyle en C₁ à C₄)carbonyle ; formyle ; alkylsulfonyle en C₁ à C₆ ; ou aminocarbonyle dans lequel le groupe amino est facultativement substitué avec un substituant (alkoxy en C₁ à C₄)carbonyle, (alkyle en C₁ à C₄)carbonyle, (alcényle en C₂ à C₄)oxycarbonyle, (alcényle en C₂ à C₄)carbonyle, alkyle en C₁ à C₄ ou alcényle en C₂ à C₄ et est en outre facultativement substitué avec un substituant alkyle en C₁ à C₄ ou alcényle en C₂ à C₄ ;
chaque indice x est indépendamment égal à 0, 1 ou 2 ; U représente un groupe CO, SO₂ et CH₂ ; ou
R⁴ représente un groupe -X^{1a}-X^{2a}-X^{3a}-X^{4a} dans lequel :
X^{1a} représente un groupe CH₂, CO ou SO₂ ;
X^{2a} représente un groupe CR^{14a}R^{15a} ;
X^{3a} représente un groupe NR^{13a}, O, S, SO₂ ou CR^{14a}R^{15a} ; dans lequel :
chacun de R^{14a} et R^{15a} est choisi indépendamment parmi les groupes énumérés ci-dessus pour R¹⁴ et R¹⁵, sous réserve que R^{14a} et R^{15a} sur le même atome de carbone ne soient choisis l'un et l'autre entre un groupe hydroxy facultativement substitué et amino facultativement substitué ; ou bien
R^{14a} et R^{15a}, conjointement, représentent un groupe oxo ;
R^{13a} représente un atome d'hydrogène ; un groupe trifluorométhyle ; alkyle en C₁ à C₆ ; alcényle en C₂ à C₆ ; (alkoxy en C₁ à C₆) carbonyle ; (alkyle en C₁ à C₆)carbonyle ; ou aminocarbonyle dans lequel le groupe amino est facultativement substitué avec un substituant (alkoxy en C₁ à C₆)carbonyle, (alkyle en C₁ à C₆)carbonyle, (alcényle en C₂ à C₆)oxycarbonyle, (alcényle en C₂ à C₆)carbonyle, alkyle en C₁ à C₆ ou alcényle en C₂ à C₆ et en outre facultativement substitué avec un substituant alkyle en C₁ à C₆ ou alcényle en C₂ à C₆ ; ou bien
deux groupes R^{14a} ou un groupe R^{13a} et R^{14a} sur des atomes adjacents, représentent conjointement une liaison, et les groupes R^{13a}, R^{14a} et R^{15a} restants répondent aux définitions précitées ; ou bien
deux groupes R^{14a} et deux groupes R^{15a} sur des atomes adjacents représentent conjointement des liaisons de sorte que X^{2a} et X^{3a} sont triplement liés ;
X^{4a} représente un groupe phényle ou un hétérocycle penta- ou hexagonal aromatique monocyclique, lié à C ou N, contenant jusqu'à quatre hétéroatomes choisis entre O, S et N et :
facultativement C-substitués avec jusqu'à trois groupes choisis entre des groupes alkylthio en C₁ à C₄ ; halogéno ; carboxy(alkyle en C₁ à C₄) ; halogénalkoxy en C₁ à C₄ ; halogénalkyle en C₁ à C₄ ; alkyle en C₁ à C₄ ; alcényle en C₂ à C₄ ; (alkoxy en C₁ à C₄)carbonyle ; formyle ; (alkyle en C₁ à C4) carbonyle ; (alcényle en C₂ à C₄)oxycarbonyle ; (alcényle en C₂ à C₄)carbonyle ; (alkyle en C₁ à C₄)carbonyloxy ; (alkoxy en C₁ à C₄)carbonyl(alkyle en C₁ à C₄) ; hydroxy ; hydroxyalkyle en C₁ à C₄ ; mercaptoalkyle en C₁ à C₄ ; alkoxy en C₁ à C₄ ; nitro ; cyano ; carboxy ; amino ou aminocarbonyle facultativement substitué comme pour les substituants correspondants dans R³ ; alkylsulfonyle en C₁ à C₄ ; alcénylsulfonyle en C₂ à C₄ ; ou aminosulfonyle dans lequel le groupe amino est facultativement substitué avec un substituant alkyle en C₁ à C₄ ou alcényle en C₂ à C₄ ; aryle ; aryl (alkyle en C₁ à C₄) ; ou aryl (alkoxy en C₁ à C₄) ; et
facultativement N-substitué avec un groupe trifluorométhyle ; alkyle en C₁ à C₄ facultativement substitué avec un substituant hydroxy, alkoxy en C₁ à C₆, alkylthio en C₁ à C₆, halogéno ou trifluorométhyle ; alcényle en C₂ à C₄ ; aryle ; aryl (alkyle en C₁ à C₄) ; (alkoxy en C₁ à C₄)carbonyle ; (alkyle en C₁ à C₄)-carbonyle ; formyle ; alkylsulfonyle en C₁ à C₆ ; ou aminocarbonyle dans lequel le groupe amino est facultativement substitué avec un substituant (alkoxy en C₁ à C₄)carbonyle, (alkyle en C₁ à C₄)carbonyle, (alcényle en C₂ à C₄)oxycarbonyle, (alcényle en C₂ à C₄)carbonyle, alkyle en C₁ à C₄ ou alcényle en C₂ à C₄, et est en outre facultativement substitué avec un substituant alkyle en C₁ à C₄ ou alcényle en C₂ à C₄ ;
n est égal à 0 ou 1 et AB représente un groupe NR¹¹CO, CONR¹¹, CO-CR⁸R⁹, CR⁶R⁷⁻CO, O-CR⁸R⁹, CR⁶R⁷⁻O, NHR¹¹-CR⁸R⁹, CR⁶R⁷⁻NHR¹¹, NR¹¹SO₂, CR⁶R⁷⁻SO₂ ou CR⁶R⁷⁻CR⁸R⁹,
sous réserve que si n=0, B ne représente pas NR¹¹, O ou SO₂,
et sous réserve que R⁶ et R⁷, et R⁸ et R⁹ ne représentent pas l'un et l'autre un groupe hydroxy ou amino facultativement substitué ;
et dans laquelle :
chacun de R⁶, R⁷, R⁸ et R⁹ est choisi indépendamment entre : H ; des groupes alkoxy en C₁ à C₆ ; alkylthio en C₁ à C₆ ; halogéno ; trifluorométhyle ; azido ; alkyle en C₁ à C₆ ; alcényle en C₂ à C₆ ; (alkoxy en C₁ à C₆)carbonyle ; (alkyle en C₁ à C₆)carbonyle ; (alcényle en C₂ à C₆)-oxycarbonyle ; (alcényle en C₂ à C₆)carbonyle ; hydroxy, amino ou aminocarbonyle facultativement substitué pour les substituants correspondants dans R³ ; alkylsulfonyle en C₁ à C₆ ; alcénylsulfonyle en C₂ à C₆ ; ou aminosulfonyle en C₁ à C₆ dans lequel le groupe amino est facultativement substitué avec un substituant alkyle en C₁ à C₆ ou alcényle en C₂ à C₆ ;
ou bien R⁶ et R⁸, conjointement, représentent une liaison et R⁷ et R⁹ répondent aux définitions précitées ;
dans le groupe amino facultativement substitué, le groupe amino est facultativement mono- ou disubstitué avec un substituant (alkoxy en C₁ à C₆)carbonyle, (alkyle en C₁ à C₆)carbonyle, (alcényle en C₂ à C₆)oxycarbonyle, (alcényle en C₂ à C₆)carbonyle, alkyle en C₁ à C₆, alcényle en C₂ à C₆, alkylsulfonyle en C₁ à C₆, alcénylsulfonyle en C₂ à C₆ ou aminocarbonyle dans lequel le groupe amino est facultativement substitué avec un substituant alkyle en C₁ à C₆ ou alcényle en C₂ à C₆ ;
dans le groupe aminocarbonyle facultativement substitué, le groupe amino est facultativement substitué avec un substituant alkyle en C₁ à C₆, hydroxyalkyle en C₁ à C₆, aminocarbonyl (alkyle en C₁ à C₆), alcényle en C₂ à C₆, (alkoxy en C₁ à C₆)carbonyle, (alkyle en C₁ à C₆)carbonyle, (alcényle en C₂ à C₆)oxycarbonyle ou (alcényle en C₂ à C₆)carbonyle, et facultativement substitué en outre avec un substituant alkyle en C₁ à C₆, hydroxyalkyle en C₁ à C₆, aminocarbonyl(alkyle en C₁ à C₆) ou alcényle en C₂ à C₆ ;
et chaque groupe R¹¹ représente indépendamment H ; un groupe trifluorométhyle ; alkyle en C₁ à C₆ ; alcényle en C₂ à C₆ ; (alkoxy en C₁ à C₆)carbonyle ; (alkyle en C₁ à C₆)carbonyle ; ou aminocarbonyle dans lequel le groupe amino est facultativement substitué avec un substituant (alkoxy en C₁ à C₆) carbonyle, (alkyle en C₁ à C₆) carbonyle, (alcényle en C₂ à C₆) oxycarbonyle, (alcényle en C₂ à C₆) carbonyle, alkyle en C₁ à C₆ ou alcényle en C₂ à C₆, et en outre facultativement substitué avec un substituant alkyle en C₁ à C₆ ou alcényle en C₂ à C₆ ;
ou bien, lorsque l'un de R⁶, R⁷, R⁸ et R⁹ contient un groupe carboxy, ces groupes peuvent former, conjointement avec R³, une liaison ester cyclique.

2. Composé suivant la revendication 1, dans lequel Z⁵ représente un groupe CH, Z³ représente CH ou CF, Z¹ représente CH ou C-OCH₃ et Z² et Z⁴ représentent chacun CH, ou bien Z¹ représente N, Z³ représente CH ou CF et Z², Z⁴ et Z⁵ représentent chacun un groupe CH.

3. Composé suivant l'une quelconque des revendications précédentes, dans lequel R¹ représente un groupe méthoxy ou fluoro et R^{1a} représente H ou, lorsque Z³ représente un groupe CR^{1a}, il peut représenter un groupe C-F.

4. Composé suivant l'une quelconque des revendications précédentes, dans lequel R² représente un atome d'hydrogène.

5. Composé suivant l'une quelconque des revendications précédentes, dans lequel R³ représente un groupe hydroxy.

6. Composé suivant l'une quelconque des revendications précédentes, dans lequel n est égal à 0 et A représente un groupe CHOH ou CH₂ et B représente un groupe CH₂, ou bien A représente un groupe NH et B représente un groupe CO, et AB(CH₂)ₙ et NR²R⁴ sont en configuration trans.

7. Composé suivant l'une quelconque des revendications précédentes, dans lequel R⁴ représente un groupe -U-R⁵₂, le groupe -U- est un groupe -CH₂-, et R⁵₂ représente un noyau hétérocyclique aromatique (A) ayant 8 à 11 atomes dans le noyau y compris 2 à 4 hétéroatomes dont au moins l'un est un atome de N ou un groupe NR¹³, ou bien le noyau hétérocyclique (A) a un noyau (a) aromatique choisi entre des groupes benzo et.pyrido facultativement substitués et le noyau (b) non aromatique et Y² a 3 à 5 atomes y compris NR¹³, O ou S liés à X⁵ et un groupe NHCO lié par N à X³, ou O lié à X³.

8. Composé suivant l'une quelconque des revendications 1 à 6, dans lequel R⁵₂ est choisi entre :
benzo[1,2,5]thiadiazole-5-yle
4H-benzo[1,4]thiazine-3-one-6-yle
2,3-dihydro-benzo[1,4]dioxine-6-yle
benzo[1,2,3]thiadiazole-5-yle
3-oxo-3,4-dihydro-2H-benzo[1,4]oxazine-6-yle
7-fluoro-3-oxo-3,4-dihydro-2H-benzo[1,4]oxazine-6-yle
2-oxo-2,3-dihydro-1H-pyrido[2,3-b]thiazine-7-yle
2,3-dihydro-[1,4]dioxino[2,3-c]pyridine-7-yle
3-oxo-3,4-dihydro-2H-pyrido[3,2-b]oxazine-6-yle
[1,2,3]thiadiazolo[5,4-b]pyridine-6-yle
3-oxo-3,4-dihydro-2*H*-pyrido[3,2-*b*]thiazine-6-yle
7-chloro-3-oxo-3,4-dihydro-2*H*-pyrido[3,2-*b*][1,4]thiazine-6-yle
7-fluoro-3-oxo-3,4-dihydro-2*H*-pyrido[3,2-*b*][1,4]thiazine-6-yle
2-oxo-2,3-dihydro-1*H*-pyrido[3,4-*b*][1,4]thiazine-7-yle.

9. Composé choisi entre:
(6-méthoxy-[1,5]naphtyridine-4-yl)-amide d'acide (1*R*,4*S*)-1-hydroxy-4-[(3-oxo-3,4-dihydro-2*H*-pyrido[3,2-*b*][1,4]-thiazine-6-ylméthyl)-amino]cyclohex-2-ènecarboxylique et
(6-méthoxy-[1,5]naphtyridine-4-yl)-amide d'acide (1*S*,4*R*)-1-hydroxy-4-(3-oxo-3,4-dihydro-2*H*-pyrido[3,2-*b*][1,4]-thiazine-6-ylméthyl)-amino]cyclohex-2-ènecarboxylique
(6-méthoxy-[1,5] naphtyridine-4-yl)-amide d' acide (1*R*,4*S*)-1-hydroxy-4-[(3-oxo-3,4-dihydro-2*H*-pyrido[3,2-*b*]-[1,4]oxazine-6-ylméthyl)-amino]cyclohex-2-ènecarboxylique et
(6-méthoxy-[1,5]naphtyridine-4-yl)-amide d'acide (1*S*,4*R*)-1-hydroxy-4-[(3oxo-3,4--dihydro-2H-pyrido[3,2-*b*]-[1,4]oxazine-6-ylméthyl)-amino]cyclohex-2-ènecarboxylique
(6-méthoxy-[1,5]naphtyridine-4-yl)-amide d'acide 1-hydroxy-*t*-4- [(2,3-dihydro[1,4dioxino[2,3-c]pyridine-7-ylméthyl)-amino]-*r*-cyclohex-2-ènecarboxylique (E2 isomère) ou un de ses dérivés pharmaceutiquement acceptables.

10. Utilisation d'un composé suivant la revendication 1 dans la production d'un médicament pour l'utilisation dans le traitement d'infections bactériennes chez des mammifères.

11. Composition pharmaceutique comprenant un composé suivant la revendication 1 et un support pharmaceutiquement acceptable.

12. Procédé pour la préparation d'un composé suivant la revendication 1, qui comprend la réaction d'un composé de formule (IV) avec un composé de formule (V) : 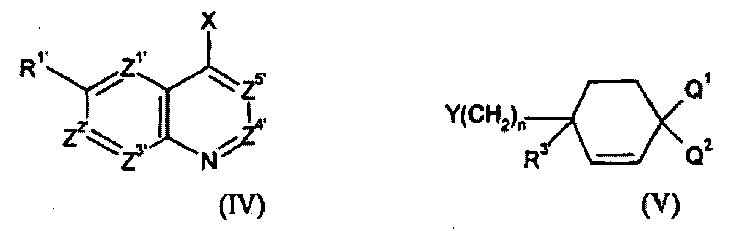 formules dans lesquelles n est tel que défini pour la formule (I) ; Z^{1'}, Z^{2'}, Z^{3'}, Z^{4'}, Z^{5'}, R^{1'} et R^{3'} représentent Z¹, Z², Z³, Z⁴, Z⁵, R¹ et R³ tels que définis pour la formule (I) ou des groupes pouvant être convertis en ceux-ci ;
Q¹ représente NR^{2'}R^{4'} ou un groupe convertible en celui-ci, où R^{2'} et R^{4'} représentent R² et R⁴ comme définis pour la formule (I) ou des groupes pouvant être convertis en ceux-ci et Q² représente H ou R^{3'}, ou bien Q¹ et Q², conjointement, forment un groupe oxo facultativement protégé ;
et X et Y peuvent représenter les associations suivantes :
(i) un de X et Y représente un groupe CO₂R^{y} et l'autre représente un groupe CH₂CO₂R^{x} ;
(ii) X représente un groupe CHR⁶R⁷ et Y représente un groupe C(=O)R⁹ ;
(iii) X représente un groupe CR⁷=PR^{z}₃ et Y représente un groupe C(=O)R⁹ ;
(iv) X représente un groupe C(=O)R⁷ et Y représente un groupe CR⁹=PR^{z}₃ ;
(v) un de Y et X représente un groupe COW et l'autre représente un groupe NHR^{11'} ;
(vi) X représente un groupe NHR^{11'} et Y représente un groupe C(=O)R⁸, ou bien X représente un groupe C(=O)R⁶ et Y représente un groupe NHR^{11'} ;
(vii) X représente un groupe NHR^{11'} et Y représente un groupe CR⁸R⁹W ;
(viii) X un groupe W ou OH et Y représente un groupe CH₂OH ;
(ix) X représente un groupe NHR^{11'} et Y représente un groupe SO₂W ;
(x) un de X et Y représente un groupe (CH₂)ₚ-W et l'autre représente un groupe (CH₂)_{q}NHR^{11'}, (CH₂)_{q}OH, (CH₂)_{q}SH ou (CH₂)_{q}SCOR^{x} dans lequel p+q=1 ;
(xi) un de X et Y représente un groupe OH et l'autre représente un groupe -CH=N₂ ;
(xii) X représente un groupe W et Y représente un groupe CONHR¹¹ ;
(xiii) X représente un groupe W et Y représente un groupe -C≡CH, suivi de la réaction sélective du groupe -C=C-intermédiaire ;
où W représente un groupe partant, par exemple halogène ou imidazolyle ; R^{x} et R^{y} représentent des groupes alkyle en C₁ à C₆ ; R^{z} représente un groupe aryle ou alkyle en C₁ à C₆ ; A' et NR^{11'} représentent des groupes A et NR¹¹ comme définis pour la formule (I), ou des groupes pouvant être convertis en ceux-ci ; et l'oxiranne est de formule : 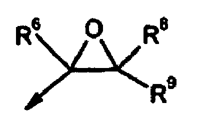 dans laquelle R⁶, R⁸ et R⁹ sont tels que définis pour la formule (I) ;
et ensuite, facultativement ou nécessairement, la conversion de Q¹ et Q² en un groupe NR^{2'}R^{4'} ; la conversion de A', Z^{1'}, Z^{2'}, Z^{3'}, Z^{4'}, Z^{5'}, R^{1'}, R^{2'}, R^{3'}, R^{4'} et NR^{11'} en A, Z¹, Z², Z³, Z⁴, Z⁵, R¹, R², R³, R⁴ et NR¹¹ ; la conversion de A-B en un autre groupe A-B, l'interconversion de R^{v}, R^{w}, R¹, R², R³ et/ou R⁴, et/ou la formation d'un dérivé pharmaceutiquement acceptable du composé obtenu.

13. Composé de formule (VII) : 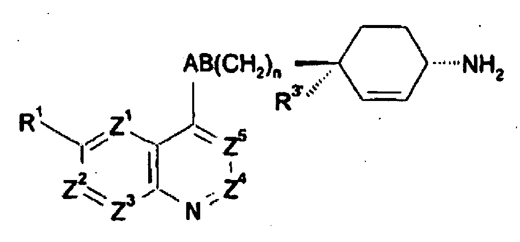 dans laquelle les variables sont telles que définies pour la formule (I) dans la revendication 1.
